(19) **Europäisches Patentamt / European Patent Office / Office européen des brevets**

(11) **EP 4 454 562 B1**

(12) # EUROPEAN PATENT SPECIFICATION

(45) Date of publication and mention
of the grant of the patent:
**03.09.2025  Bulletin 2025/36**

(21) Application number: **23851293.3**

(22) Date of filing: **12.05.2023**

(51) International Patent Classification (IPC):
**A61B 5/346** (2021.01)     **A61B 5/00** (2006.01)
**A61B 5/332** (2021.01)

(52) Cooperative Patent Classification (CPC):
**A61B 5/332; A61B 5/346; A61B 5/681;
A61B 5/7203; A61B 5/725**

(86) International application number:
**PCT/CN2023/093992**

(87) International publication number:
**WO 2024/032063 (15.02.2024 Gazette 2024/07)**

(54) **ELECTROCARDIOGRAM SIGNAL PROCESSING METHOD AND ELECTRONIC DEVICE**

ELEKTROKARDIOGRAMMSIGNALVERARBEITUNGSVERFAHREN UND ELEKTRONISCHE VORRICHTUNG

PROCÉDÉ DE TRAITEMENT DE SIGNAL D'ÉLECTROCARDIOGRAMME ET DISPOSITIF ÉLECTRONIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **09.08.2022  CN 202210948840**

(43) Date of publication of application:
**30.10.2024  Bulletin 2024/44**

(73) Proprietor: **Honor Device Co., Ltd.
Shenzhen, Guangdong 518040 (CN)**

(72) Inventors:
• **YIN, Haibo
Shenzhen, Guangdong 518040 (CN)**
• **ZHANG, Xiaofan
Shenzhen, Guangdong 518040 (CN)**

(74) Representative: **Goddar, Heinz J.
Boehmert & Boehmert
Anwaltspartnerschaft mbB
Pettenkoferstrasse 22
80336 München (DE)**

(56) References cited:
WO-A1-2022/014902     CN-A- 111 345 808
CN-A- 112 674 776     CN-A- 114 521 899
US-A1- 2013 237 874     US-A1- 2013 245 478
US-A1- 2019 384 757

• LIU BAOYING ET AL: "ECG signal denoising based on similar segments cooperative filtering", BIOMEDICAL SIGNAL PROCESSING AND CONTROL, ELSEVIER, AMSTERDAM, NL, vol. 68, 19 May 2021 (2021-05-19), XP086638929, ISSN: 1746-8094, [retrieved on 20210519], DOI: 10.1016/J.BSPC.2021.102751

## Description

This application claims priority to Chinese Patent Application No. 202210948840.5, filed with the China National Intellectual Property Administration on August 9, 2022 and entitled "ELECTROCARDIOGRAM SIGNAL PROCESSING METHOD AND ELECTRONIC DEVICE'.

## TECHNICAL FIELD

This application relates to the field of terminal technologies, and in particular, to an electrocardiogram signal processing method and an electronic device.

## BACKGROUND

With development of terminal technologies, wearable devices are gradually applied to people's work and life. To meet a growing requirement of a user for health management of the user, the wearable device may provide a plurality of user health data detection services, such as heart rate monitoring, sleep monitoring, and electrocardiogram signal monitoring.

Generally, an electrocardiogram signal is weak. In a process in which the wearable device collects the electrocardiogram signal, the wearable device is susceptible to interference from factors such as body motion, respiration, and electromagnetic interference in an environment. Consequently, noise exists in the electrocardiogram signal collected by the wearable device.

In a possible implementation, the wearable device may use a filter to filter out a part of noise, for example, baseline drift and power frequency interference. However, the electrocardiogram signal output by the wearable device is still not accurate. US 2013/245478 A1 and LIU BAOYING ET AL, "ECG signal denoising based on similar segments cooperative filtering", BIOMEDICAL SIGNAL PROCESSING AND CONTROL, ELSEVIER, AMSTERDAM, NL, vol. 68, 19 May 2021 (2021-05-19), ISSN: 1746-8094, DOI: 10.1016/ J.BSPC.2021.102751, relate to ECG signal filtering.

## SUMMARY

Embodiments of this application provide an electrocardiogram signal processing method and an electronic device, so that interference of noise to an electrocardiogram signal can be reduced by dividing electrocardiogram data into areas and performing different smoothing processing in different areas.

According to a first aspect, an embodiment of this application provides an electrocardiogram signal processing method, applied to an electronic device, where the method includes: The electronic device preprocesses electrocardiogram data to obtain first data; the electronic device divides data points in the first data into data points in a first-type area, data points in a second-type area, and data points in a buffer-type area; the electronic device performs smoothing processing on the data points in the second-type area by using a plurality of fixed-length windows, to obtain second data; the electronic device performs smoothing processing on data points in a third-type area by using a plurality of fixed-length windows, to obtain third data; the electronic device performs smoothing processing on data points in a fourth-type area by using a plurality of non-fixed-length windows, to obtain fourth data; and the electronic device integrates the second data, the third data, the fourth data, and the data points in the first-type area, to obtain fifth data obtained after the electrocardiogram data is smoothed. In this way, the electronic device performs smoothing processing on the data points in the smooth second-type area and the data points in the smooth third-type area by using fixed-length sliding windows, and the electronic device performs smoothing processing on the data points in the fourth-type area by using variable-length sliding windows, thereby performing smoothing processing on noise in an electrocardiogram signal, and reducing interference of noise to the electrocardiogram signal.

In a possible implementation, when the fourth-type area is an area that transitions from the first-type area to the third-type area, lengths of the plurality of non-fixed-length windows gradually increase over time; or when the fourth-type area is an area that transitions from the third-type area to the first-type area, lengths of the plurality of non-fixed-length windows gradually decrease over time. In this way, the electronic device may set the lengths of the non-fixed-length windows based on features of data points between the first-type area and the third-type area, thereby improving a smoothing effect on the data points in the fourth-type area.

In a possible implementation, first variability is related to determining the data point in the first-type area, the data point in the third-type area, and the data point in the fourth-type area, and that the electronic device divides data points in the first data into data points in a first-type area, data points in a second-type area, data points in a third-type area, and data points in a fourth-type area includes: The electronic device calculates the first variability of the data point in the first data; and when the first variability is less than a first variability threshold, the electronic device determines that the data point in the first data is the data point in the second-type area; or when the first variability is not less than the first variability threshold, the electronic device determines that the data point in the first data is the data point in the first-type area, the data point in the third-type area, or the data point in the fourth-type area. In this way, the electronic device may obtain the second-type area and another area through division based on the first variability, so that the electronic device subsequently performs area-based smoothing processing on the electrocardiogram data.

In a possible implementation, second variability is related to determining the data point in the first-type area, and that the electronic device divides data points in the first data into data points in a first-type area, data points in a second-type area, data points in a third-type area, and data points in a fourth-type area further includes: The electronic device calculates the second variability of the data point in the first-type area, the data point in the third-type area, or the data point in the fourth-type area; and when the second variability is not less than a second variability threshold, the electronic device determines that the data point in the first data is the data point in the first-type area; or when the second variability is less than the second variability threshold, the electronic device determines that the data point in the first data is the data point in the third-type area or the data point in the fourth-type area. In this way, the electronic device may obtain the first-type area and the buffer-type area through division based on the second variability, so that the electronic device subsequently performs area-based smoothing processing on the electrocardiogram data.

In a possible implementation, the first variability $M_1$ meets the following formula: $M_1$=mean(abs(diff($X_1$)))/(2k+1), where k is a positive integer, a window length selected when $M_1$ is calculated is 2k+2, and $X_1$ is a set of 2k+2 data points in the first data in a window; and the second variability $M_2$ meets the following formula: $M_2$=mean(abs(diff($X_2$)))/(2j+1), where j is a positive integer, a window selected when $M_2$ is calculated is 2j+2, and $X_2$ is a set of 2j+2 data points in the first data in the window. In this way, the electronic device may obtain an area through division based on the first variability and the second variability, so that the electronic device subsequently performs area-based smoothing processing on the electrocardiogram data.

In a possible implementation, $x_t^{r_2}$ is a data point in the second data at a moment t, and the data point $x_t^{r_2}$ in the second data meets the following formula: $x_t^{r_2} = (x_{t-H}+\ldots+x_t+,\ldots,+x_{t+H})/(2H+1)$, where xt is a data point in the first data at the moment t, $x_{t-H}$ is a data point in the first data at a moment t-H, $x_{t+H}$ is a data point in the first data at a moment t+H, H is a positive integer and H is a constant, and a window length selected when $x_t^{r_2}$ is calculated is 2H+1. In this way, the electronic device may perform area-based smoothing processing on the data points in the second-type area, to obtain the second data.

In a possible implementation, the preset length is q, $x_t^{r_3}$ is a data point in the third data at a moment t, and the data point $x_t^{r_3}$ in the third data meets the following formula: $x_t^{r_3} = (x_{t-Q}+\ldots+x_t+,\ldots,+x_{t+Q})/(2Q+1)$, where $x_{t-Q}$ is a data point in the first data at a moment t-Q, $x_{t+Q}$ is a data point in the first data at a moment t+Q, Q is a positive integer and Q is a constant, Q<H, a window length selected when $x_t^{r_3}$ is calculated is 2Q+1, and a value range of the window length is [1, q]. In this way, the electronic device may perform area-based smoothing processing on the data points in the third-type area, to obtain the third data.

In a possible implementation, $x_t^{r_4}$ is a data point in the fourth data at a moment t, and the data point $x_t^{r_4}$ in the fourth data meets the following formula: $x_t^{r_4} = (x_{t-u}+\ldots+x_t+,\ldots,+x_{t+u})/(2u+1)$, where $x_{t-u}$ is a data point in the first data at a moment t-u, $x_{t+u}$ is a data point in the first data at a moment t+u, u is a positive integer and u is a variable, and a window length selected when $x_t^{r_4}$ is calculated is 2u+1. In this way, the electronic device may perform area-based smoothing processing on the data points in the fourth-type area, to obtain the fourth data.

In a possible implementation, the method further includes: When the electronic device receives a trigger operation used to start electrocardiogram data detection, the electronic device displays data points in the fifth data. In this way, the electronic device may display the electrocardiogram data obtained after noise is removed.

In a possible implementation, the electronic device includes a wearable device, and the wearable device includes one or more of the following: a smartwatch, a smart band, or smart glasses.

According to a second aspect, an embodiment of this application provides an electronic device, including a memory, a processor, and computer program stored in the memory and capable of running on the processor, where when the processor executes the computer program, the electronic device is enabled to perform the electrocardiogram signal processing method according to any one of the first aspect or the implementations of the first aspect.

According to a third aspect, an embodiment of this application provides a computer-readable storage medium, where the computer-readable storage medium stores a computer program, and when the computer program is executed by a processor, a computer is enabled to perform the electrocardiogram signal processing method according to any one of the first aspect or the implementations of the first aspect.

According to a fourth aspect, a computer program product is provided, including a computer program, where when the computer program is run, a computer is enabled to perform the electrocardiogram signal processing method according to any one of the first aspect or the implementations of the first aspect.

It should be understood that, the second aspect to the fourth aspect of this application correspond to the technical solution of the first aspect of this application, and the beneficial effects achieved by each aspect and the corresponding feasible implementations are similar. Details are not described again.

## BRIEF DESCRIPTION OF DRAWINGS

FIG. 1 is a schematic diagram of a scenario according to an embodiment of this application;
FIG. 2 is a schematic diagram of a structure of a wearable device according to an embodiment of this application;
FIG. 3 is a schematic diagram of a software structure of a smartwatch according to an embodiment of this application;
FIG. 4 is a schematic diagram of area division of an electrocardiogram signal according to an embodiment of this application;
FIG. 5 is a schematic flowchart of an electrocardiogram signal processing method according to an embodiment of this application;
FIG. 6 is a schematic diagram of a curve of variability of electrocardiogram data according to an embodiment of this application;
FIG. 7 is a schematic diagram of an scenario of an electrocardiogram signal processing method according to an embodiment of this application;
FIG. 8 is a schematic diagram of curves of first data and fifth data according to an embodiment of this application;
FIG. 9 is a schematic diagram of a structure of an electrocardiogram signal processing apparatus according to an embodiment of this application; and
FIG. 10 is a schematic diagram of a structure of an electronic device according to an embodiment of this application.

## DESCRIPTION OF EMBODIMENTS

To clearly describe technical solutions in embodiments of this application, in the embodiments of this application, words such as "first" and "second" are used to distinguish between same items or similar items with basically the same functions and effects. For example, a first value and a second value are merely intended to distinguish between different values, but not to limit a sequence thereof. A person skilled in the art may understand that the words such as "first" and "second" do not limit a quantity and an execution sequence, and the words such as "first" and "second" do not indicate a definite difference.

It should be noted that in this application, the word such as "example" or "for example" is used to represent giving an example, an illustration, or a description. Any embodiment or design solution described as an "example" or "for example" in this application should not be explained as being more preferred or having more advantages than other embodiments or design solutions. Exactly, the words such as "example" or "for example" are used to present related concepts in a specific manner.

In this application, "at least one" means one or more, and "a plurality of" means two or more. "And/or" describes an association relationship between associated objects, and represents that three relationships may exist. For example, A and/or B may represent the following cases: Only A exists, both A and B exist, and only B exists, where A and B may be singular or plural. The character "/" usually indicates an "or" relationship between associated objects. "At least one of the following items" or a similar expression thereof means any combination of these items, including a single item or any combination of a plurality of items. For example, at least one of a, b, or c may represent a, b, c, a and b, a and c, b and c, or a, b, and c, where a, b, and c may be singular or plural.

To meet a growing requirement of a user for health management of the user, the wearable device may provide a plurality of user health data detection services, such as heart rate monitoring, sleep monitoring, and electrocardiogram signal monitoring. Generally, an electrocardiogram signal is weak. In a process in which the wearable device collects the electrocardiogram signal, the wearable device is susceptible to interference from factors such as body motion, respiration, and electromagnetic interference in an environment. Consequently, noise exists in the electrocardiogram signal collected by the wearable device.

In a possible implementation, the wearable device may use a filter to filter out a part of noise, for example, baseline drift and power frequency interference. However, interference of much noise (such as electromyographic noise and motion noise) exists in an entire frequency band. The noise is weak and difficult to eliminate. For the electrocardiogram signal, the noise has a more obvious impact on a weak and steady segment of the electrocardiogram signal. Consequently, accuracy of the electrocardiogram signal is reduced.

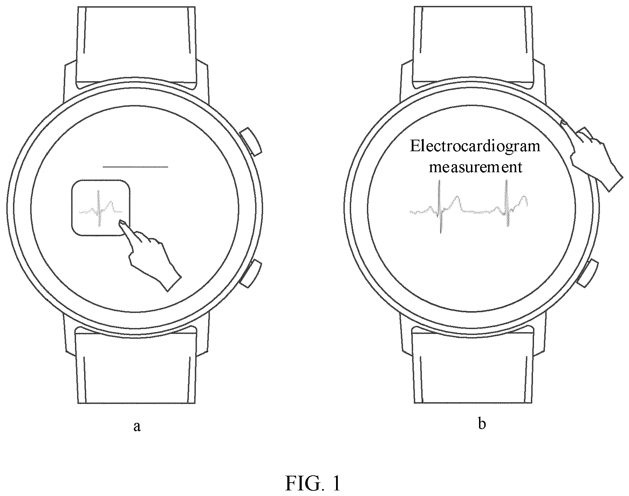

For example, FIG. 1 is a schematic diagram of a scenario of an electrocardiogram signal processing method according to an embodiment of this application. As shown in FIG. 1:

An electronic device may be configured with an "electrocardiography" application program, and the application program may support the electronic device in measuring an electrocardiogram signal of a user. The electronic device may display an interface a in FIG. 1. The interface a in FIG. 1 may include an "electrocardiography" icon. When the electronic device detects a touch control operation on the "electrocardiography" icon, the terminal device starts the "electrocardiography" application program. The user may use an electrocardiogram monitoring service. The electronic device enters an interface shown in b in FIG. 1. The interface b in FIG. 1 may display, in real time, an electrocardiogram signal collected and processed by the electronic device. The user can intuitively view the electrocardiography. In some embodiments, the electronic device may be a smartwatch, and the smartwatch may establish a connection to an electronic device such as a mobile phone. In this way, the user may know a health status of the user, and proper health guidance may also be provided for the user.

In a possible implementation, the electronic device collects an original electrocardiogram signal, and performs filtering processing on the original electrocardiogram signal by using a filter, to filter out a part of noise (for example, baseline drift and power frequency interference), and display the processed electrocardiogram signal on the electronic device. However, for a part of noise (such as electromyographic noise and motion noise), it is difficult for the filter to eliminate the noise. The electrocardiogram signal is interfered with by the part of noise. This may cause distortion of an electrocardiogram track.

In view of this, the embodiments of this application provide an electrocardiogram signal processing method. An electronic device preprocesses electrocardiogram data to obtain first data; the electronic device divides data points in the first data into data points in a first-type area, data points in a second-type area, and data points in a buffer-type area; the electronic device performs smoothing processing on the data points in the second-type area by using a plurality of fixed-length windows, to obtain second data; the electronic device performs smoothing processing on data points in a third-type area by using a plurality of fixed-length windows, to obtain third data; the electronic device performs smoothing processing on data points in a fourth-type area by using a plurality of non-fixed-length windows, to obtain fourth data; and the electronic device integrates the second data, the third data, the fourth data, and the data points in the first-type area, to obtain fifth data obtained after the electrocardiogram data is smoothed. In this way, the electronic device performs smoothing processing on the data points in the smooth second-type area and the data points in the smooth third-type area by using fixed-length sliding windows, and the electronic device performs smoothing processing on the data points in the fourth-type area by using variable-length sliding windows, thereby performing smoothing processing on noise in an electrocardiogram signal, and reducing interference of noise to the electrocardiogram signal.

It may be understood that, an example in which the electronic device is a smartwatch among wearable devices is used for description in the embodiments of this application, and this example does not constitute a limitation on the embodiments of this application.

It may be understood that, the electronic device in the embodiments of this application may support measurement of electrocardiography, and the electronic device may include a mobile phone (mobile phone), a smart TV, a wearable device, a tablet computer (Pad), a computer having a wireless sending and receiving function, a virtual reality (virtual reality, VR) terminal device, an augmented reality (augmented reality, AR) device, a wireless device in industrial control (industrial control), or a wireless device in self-driving (self-driving). The wearable device may include devices such as a smartwatch, a smart band, smart gloves, smart glasses, a virtual reality (virtual reality, VR) terminal device, or a smart waistband.

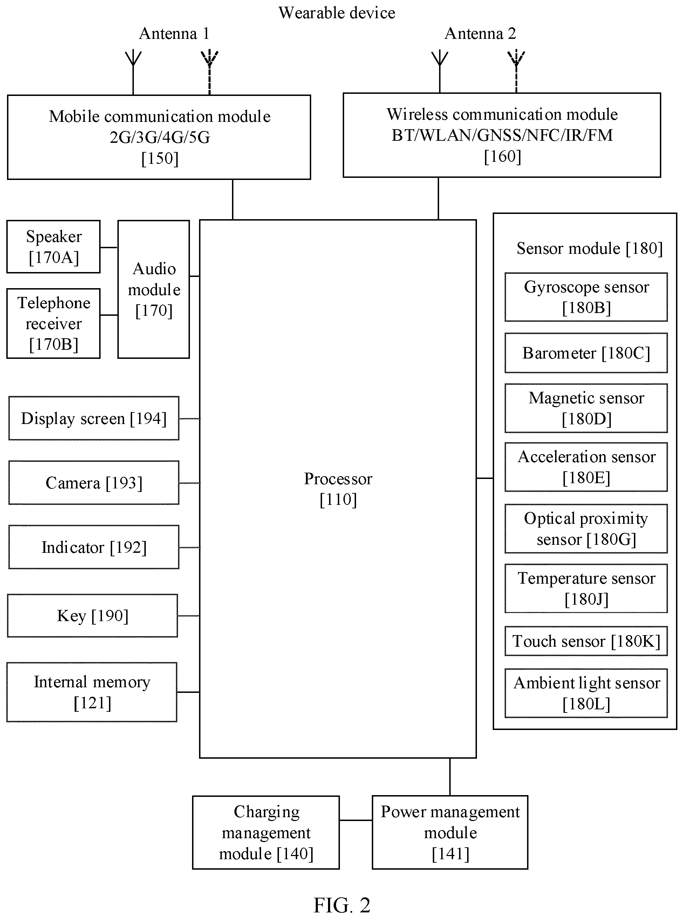

Specific forms of the electronic device and the wearable device are not specifically limited in the embodiments of this application. For a better understanding of the embodiments of this application, a structure of the wearable device in the embodiments of this application is described below. For example, FIG. 2 is a schematic diagram of a structure of a wearable device according to an embodiment of this application.

The wearable device may include a processor 110, an internal memory 121, a universal serial bus (universal serial bus, USB) interface, a charging management module 140, a power management module 141, an antenna 1, an antenna 2, a mobile communication module 150, a wireless communication module 160, an audio module 170, a speaker 170A, a telephone receiver 170B, a sensor module 180, a key 190, an indicator 192, a camera 193, a display screen 194, and the like. The sensor module 180 may include a gyroscope sensor 180B, a barometer 180C, a magnetic sensor 180D, an acceleration sensor 180E, an optical proximity sensor 180G, a temperature sensor 180J, a touch sensor 180K, an ambient light sensor 180L, and the like.

It may be understood that the structure illustrated in this embodiment of this application does not constitute a specific limitation on the wearable device. In some other embodiments of this application, the wearable device may include more or fewer components than those shown in the figure, or combine some components, or split some components, or have different component arrangements. The components shown in the figure may be implemented by using hardware, software, or a combination of software and hardware.

The processor 110 may include one or more processing units. Different processing units may be independent devices, or may be integrated into one or more processors. A memory may be further disposed in the processor 110, and is configured to store instructions and data.

The charging management module 140 is configured to receive charging input from a charger. The charger may be a wireless charger or a wired charger. The power management module 141 is configured to be connected to the charging management module 140 and the processor 110.

A wireless communication function of the wearable device may be implemented by using the antenna 1, the antenna 2, the mobile communication module 150, the wireless communication module 160, a modem processor, a baseband processor, and the like.

The antenna 1 and the antenna 2 are configured to transmit and receive an electromagnetic wave signal. The antenna in the wearable device may be configured to cover one or more communication bands. Different antennas may be further multiplexed to increase antenna utilization.

The mobile communication module 150 may provide a solution that is applied to the wearable device and that includes wireless communication such as 2G/3G/4G/5G. The mobile communication module 150 may include at least one filter, a switch, a power amplifier, a low noise amplifier (low noise amplifier, LNA), and the like. The mobile communication module 150 may receive an electromagnetic wave by using the antenna 1, perform processing such as filtering or amplification on the received electromagnetic wave, and transmit a processed electromagnetic wave to the modem processor for demodulation.

The wireless communication module 160 may provide a solution that is applied to the wearable device and that includes wireless communication such as a wireless local area network (wireless local area networks, WLAN) (for example, a wireless fidelity (wireless fidelity, Wi-Fi) network), Bluetooth (bluetooth, BT), a global navigation satellite system (global navigation satellite system, GNSS), and frequency modulation (frequency modulation, FM).

The wearable device implements a display function by using a GPU, the display screen 194, an application processor, and the like. The GPU is a microprocessor for image processing and is connected to the display screen 194 and the application processor. The GPU is configured to perform mathematical and geometric calculation for graphics rendering.

The display screen 194 is configured to display an image, a video, and the like. The display screen 194 includes a display panel. In some embodiments, the wearable device may include one or N display screens 194, where N is a positive integer greater than 1.

The wearable device may implement a shooting function by using an ISP, the camera 193, a video codec, the GPU, the display screen 194, the application processor, and the like.

The camera 193 is configured to capture a still image or a video. In some embodiments, the wearable device may include one or N cameras 193, where N is a positive integer greater than 1.

The internal memory 121 may be configured to store computer-executable program code, and the executable program code includes instructions. The internal memory 121 may include a program storage area and a data storage area.

The wearable device may implement an audio function by using the audio module 170, the speaker 170A, the telephone receiver 170B, the application processor, and the like, for example, music playing and audio recording.

The audio module 170 is configured to convert digital audio information into an analog audio signal for output, and is further configured to convert analog audio input into a digital audio signal. The speaker 170A, also referred to as a "loudspeaker", is configured to convert an audio electrical signal into a sound signal. The wearable device may listen to music or answer a call in a hands-free mode by using the speaker 170A. The telephone receiver 170B, also referred to as an "earpiece", is configured to convert an audio electrical signal into a sound signal. When the wearable device receives a call or a voice message, the telephone receiver 170B can be placed close to an ear to receive the voice.

The gyroscope sensor 180B may be configured to determine a motion posture of the wearable device. The magnetic sensor 180D includes a Hall sensor.

The barometer 180C is configured to measure barometric pressure. In some embodiments, the wearable device may calculate an altitude based on a barometric pressure value measured by the barometer 180C, to assist in positioning and navigation. In this embodiment of this application, the barometer 180C is configured to measure a barometric pressure situation in which the wearable device is located. For example, the barometer 180C may be configured to detect whether the wearable device is underwater.

The acceleration sensor 180E may detect magnitudes of accelerations in all directions (usually on three axes) of the wearable device. In this embodiment of this application, the acceleration sensor 180E is configured to detect whether the wearable device is in a motion state.

The optical proximity sensor 180G may be configured to detect whether an object approaches or moves away from the wearable device. The ambient light sensor 180L is configured to sense brightness of ambient light. The temperature sensor 180J is configured to detect temperature. In this embodiment of this application, the temperature sensor is configured to detect temperature of an environment in which the wearable device is located.

The touch sensor 180K is also referred to as a "touch control device". The touch sensor 180K may be disposed on the display screen 194. The touch sensor 180K and the display screen 194 form a touchscreen, also referred to as a "touch control screen".

The key 190 includes a power-on/off key, a volume key, and the like. The key 190 may be a mechanical key, or may be a touch key. The wearable device may receive key input, and generate key signal input related to user settings and function control of the wearable device. The indicator 192 may be an indicator light, may be configured to indicate a charging status or a power change, and may be further configured to indicate a message, a missed call, a notification, and the like.

A software system of the wearable device may use a layered architecture, an event-driven architecture, a microkernel architecture, a microservice architecture, a cloud architecture, or the like. In this embodiment of this application, a system with the layered architecture is used as an example to describe a software structure of the wearable device.

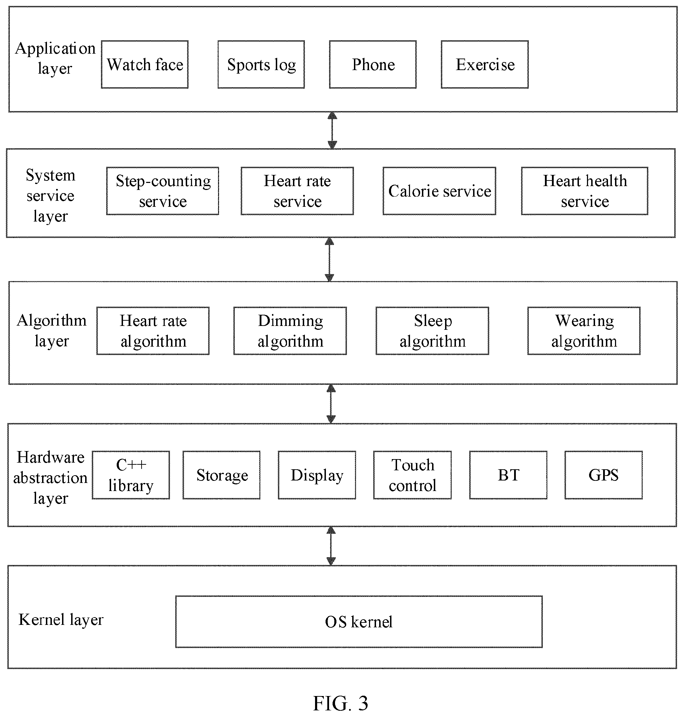

For example, the wearable device is a smartwatch. FIG. 3 is a schematic diagram of a software structure of a smartwatch according to an embodiment of this application.

As shown in FIG. 3, the layered architecture divides software into several layers, and each layer has a clear role and task. The layers communicate with each other through a software interface. In some embodiments, the software architecture of the smartwatch may be divided into five layers from top to bottom: an application (application, APP) layer, a system service layer, an algorithm layer, a hardware abstraction layer (hardware abstraction layer, HAL), and a kernel (kernel) layer.

The application layer may include a series of application programs. For example, the application programs may include a watch face application, a sports log application, a phone application, an exercise application, and the like.

The system service layer is used to provide system support for the application programs in the application layer. For example, the system service layer may include modules such as a step-counting service, a heart rate service, a calorie service, and a heart health service.

The algorithm layer is used to provide algorithm support for the system service layer. For example, the algorithm layer may include a heart rate algorithm, a dimming algorithm, a sleep algorithm, a wearing algorithm, and the like. In this embodiment of this application, the heart rate algorithm, the dimming algorithm, the sleep algorithm, and the wearing algorithm may be jointly used to support the heart rate service, to detect heart rate features of a user in different states.

In the hardware abstraction layer, all hardware-related operations required by an upper layer of the system need to invoke a HAL-related application programming interface (application programming interface, API). Some functions are specified in a software architecture layer of each hardware device, and the HAL layer may be used to implement these functions.

The hardware abstraction layer may include an interface corresponding to a C++ library, a storage interface, a display interface, a touch control interface, a Bluetooth (bluetooth) interface, a global positioning system (global positioning system, GPS) interface, and the like.

The kernel layer may be a layer between hardware and software. A kernel is system software that provides the hardware abstraction layer and a disk and functions such as file system control and multitasking. The kernel is a core of an operating system and a most fundamental part of the operating system. The kernel is responsible for managing a system process, a memory, a device driver, a file, a network system, and the like, and determines performance and stability of the system. The kernel is a part of software that provides many application programs with secure access to computer hardware. Such access is limited, and the kernel determines when and how long a program operates a part of hardware.

The kernel layer may be an operating system kernel (operating system kernel, OS kernel).

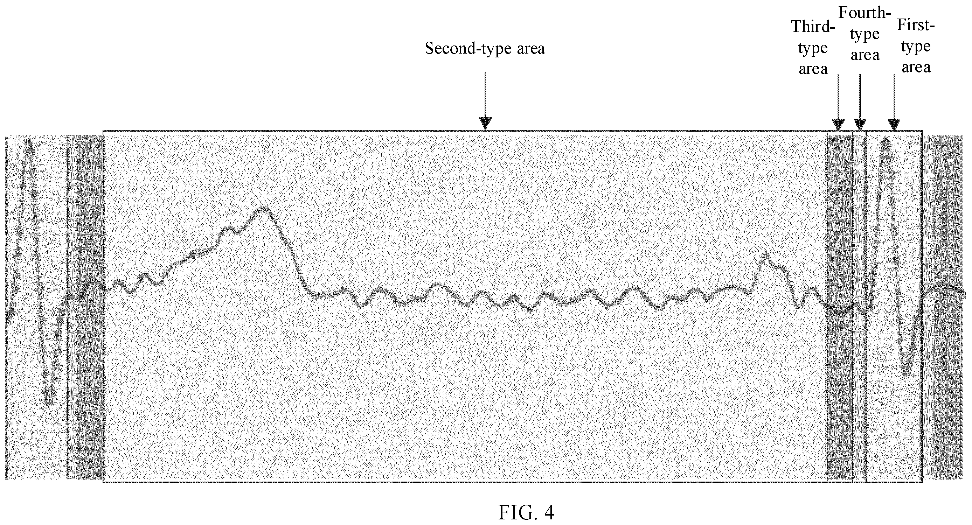

Definitions in the embodiments of this application are first described below in a form of a schematic diagram with reference to FIG. 4. FIG. 4 is a schematic diagram of area division of an electrocardiogram signal according to an embodiment of this application. As shown in FIG. 4:

In this embodiment of this application, electrocardiogram data is divided into four areas, including a first-type area, a second-type area, a third-type area, and a fourth-type area. For example, the first-type area, the second-type area, the third-type area, and the fourth-type area may be shown in FIG. 4. The first-type area may be a part of a QRS waveband in the electrocardiogram data, and the first-type area includes an R wave. The second-type area may include an area from a start point of a T wave to an end point of a P wave. It may be understood that there is a buffer-type area between the first-type area and the second-type area. The buffer-type area includes a third-type area and a fourth-type area. The third-type area is an area, with a preset length, that is in the buffer-type area and that is adjacent to a boundary of the second-type area. The fourth-type area is an area other than the third-type area in the buffer-type area.

It should be noted that, in this embodiment of this application, an n-type area may be an area formed by data points of different signal characteristic types in the electrocardiogram data, instead of a fixed area formed by data points in a specific fixed time period in the electrocardiogram data. For example, the electrocardiogram data is displayed by using time as a horizontal axis unit. The electronic device collects two groups of electrocardiogram data, and time periods in which first-type areas and second-type areas of the two groups of electrocardiogram data are located are generally different.

It may be understood that the electrocardiogram data may include a QRS waveband with a large electrocardiogram signal amplitude, and a PR waveband, an ST waveband, a T waveband, a U waveband, and the like with a small electrocardiogram signal amplitude. According to the electrocardiogram signal processing method in this embodiment of this application, based on interference degrees of noise to electrocardiogram data with different amplitudes, the first-type area may be defined as a non-suppressed area, and the second-type area may be defined as a suppressed area. The non-suppressed area may be understood as follows: Noise weakly interferes with an electrocardiogram signal with a high amplitude, and when smoothing processing is performed on this part of electrocardiogram signal, a signal feature of an original electrocardiogram signal may be lost. Therefore, in this embodiment of this application, noise in this part of the electrocardiogram signal is not suppressed. The suppressed area may be understood as follows: Noise strongly interferes with an electrocardiogram signal with a low amplitude. The electrocardiogram signal processing method in this embodiment of this application is used to suppress interference of noise to the electrocardiogram signal with a low amplitude. Suppression may be understood as reducing energy magnitude of noise. The electrocardiogram data is used as an example. Suppression may be numerically reducing magnitude of noise in the electrocardiogram data.

Further, to improve a smoothing effect on noise in the electrocardiogram data, in this embodiment of this application, data points in the buffer-type area are further divided into data points in the third-type area and data points in the fourth-type area. The third-type area is an area, with a preset length, that is in the buffer-type area and that is adjacent to a boundary of the second-type area. The fourth-type area is an area other than the third-type area in the buffer-type area. In this embodiment of this application, the electronic device divides the electrocardiogram data into data points in the first-type area, the second-type area, the third-type area, and the fourth-type area. Based on a suppression effect on noise in the electrocardiogram signal, the first-type area may be defined as a non-suppressed area, the second-type area may be defined as a strongly suppressed area, the third-type area may be defined as a weakly suppressed area, and the fourth-type area may be defined as a non-suppressed buffer area.

The following describes in detail, by using specific embodiments, the technical solutions of this application and how to resolve the foregoing technical problem in the technical solutions of this application. The following several specific embodiments may be implemented independently, or may be combined with each other. For same or similar concepts or processes, details may not be described in some embodiments.

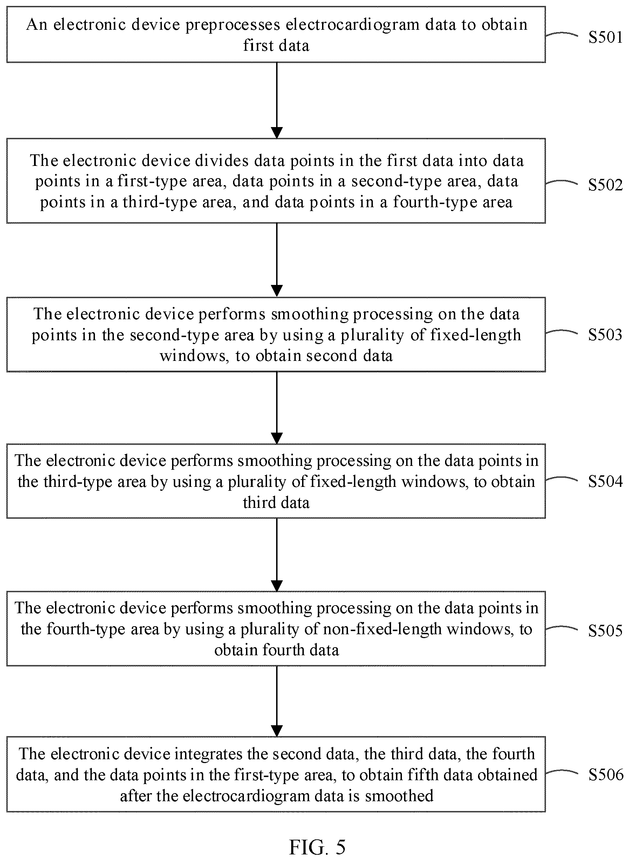

For example, FIG. 5 is a schematic flowchart of an electrocardiogram signal processing method according to an embodiment of this application. As shown in FIG. 5:

S501. An electronic device preprocesses electrocardiogram data to obtain first data, where the preprocessing is used to filter out baseline drift and power frequency interference noise in the electrocardiogram data.

In this embodiment of this application, the electronic device may perform high-pass filtering and low-pass filtering processing on the electrocardiogram data. The two types of filters generally may filter out noise outside an effective interval of an electrocardiogram signal. The noise that is filtered out may be noise of baseline drift and noise of power frequency interference.

For example, the electronic device may preprocess the electrocardiogram data by using an n-order high-pass filter, and a frequency lower limit that is set for preprocessing may be $F_l$.

The electronic device may preprocess the electrocardiogram data by using an m-order low-pass filter, and a frequency upper limit that is set for preprocessing may be $F_h$. $F_l$ and $F_h$ may be customized.

The electronic device obtains the first data obtained through filtering.

S502. The electronic device divides data points in the first data into data points in a first-type area, data points in a second-type area, and data points in a buffer-type area.

The electronic device may divide the electrocardiogram data into different areas based on amplitudes of the data points in the first data, and the different areas may include the first-type area, the second-type area, and the buffer-type area. The first-type area may be a part of a QRS waveband in the electrocardiogram data, and the first-type area includes an R wave. The second-type area may include an area from a start point of a T wave to an end point of a P wave. There is a buffer-type area between the first-type area and the second-type area. The buffer-type area includes a third-type area and a fourth-type area. The third-type area is an area, with a preset length, that is in the buffer-type area and that is adjacent to a boundary of the second-type area. The fourth-type area is an area other than the third-type area in the buffer-type area.

It may be understood that the first-type area, the second-type area, and the buffer area may appear periodically. Data points in various areas have specific features or waveforms. The electronic device may obtain various areas through division based on features of various data points, or may obtain various areas through division based on an electrocardiogram data waveform. A specific division manner of various areas is not limited in this embodiment of this application.

S503. The electronic device performs smoothing processing on the data points in the second-type area by using a plurality of fixed-length windows, to obtain second data.

The window used to process the second-type area may be a fixed-length sliding window, and the plurality of fixed-length windows may be understood as follows: At a first moment, a window with a length a uses a first data point as a center point; and at a second moment, the window with the length a uses a second data point as a center point. The electrocardiogram data is discrete data points, the first data point may be a previous data point adjacent to the second data point, and the first moment is earlier than the second moment. By analogy, windows at different moments may exhibit a similar effect that the window slides on the electrocardiogram data based on time.

The electronic device may perform smoothing processing on data points in the windows in the second-type area to obtain processed data points in the second-type area. In this embodiment of this application, the processed data points in the second-type area may be defined as the second data.

Smoothing processing may be understood as removing or processing data points forming spikes and the like, so that values of smoothed data points form a smooth curve.

S504. The electronic device performs smoothing processing on the data points in the third-type area by using a plurality of fixed-length windows, to obtain third data, where a length of a fixed window used to perform smoothing processing on the third-type area is less than a length of a fixed window used to perform smoothing processing on the second-type area.

The window used to process the third-type area may be a window with a fixed length, and a window length that is set in step S504 may be less than a window length that is set in step S503. The electronic device may perform smoothing processing on data points in the windows in the third-type area to obtain processed data points in the third-type area. In this embodiment of this application, the processed data points in the third-type area may be defined as the third data.

It may be understood that the third-type area is located in the buffer-type area between the first-type area and the second-type area. An amplitude difference between adjacent data points in the third-type area may be slightly higher than an amplitude difference between adjacent data points in the second-type area, and interference of noise to an electrocardiogram signal in the second-type area is strong. Therefore, the electronic device may select a long fixed-length window to process the electrocardiogram data, so that a better smoothing effect is achieved in the second-type area. However, an area covered by the third-type area is small, and if the electronic device selects a long window, electrocardiogram data in the third-type area may be excessively smoothed, and consequently features of a part of data points are lost. Therefore, the electronic device may smooth the electrocardiogram data in the third-type area by using a short fixed window.

S505. The electronic device performs smoothing processing on the data points in the fourth-type area by using a plurality of non-fixed-length windows, to obtain fourth data.

The window used to process the fourth-type area may be a variable-length sliding window, and the plurality of non-fixed-length windows may be understood as follows: At a first moment, a window with a length a uses a first data point as a center point; at a second moment, a window with a length b (b>a or b<a) uses a second data point as a center point; and so on.

The plurality of non-fixed-length windows may be set as follows:
In a possible implementation, when the fourth-type area is an area that transitions from the first-type area to the third-type area, lengths of the plurality of non-fixed-length windows gradually increase over time. For example, a window length a at a first moment is less than a window length b at a second moment, and the window length at the second moment is less than a window length c at a third moment. By analogy, the window length gradually increases over time.

It may be understood that an amplitude of electrocardiogram data in the first-type area is high, an amplitude of electrocardiogram data in the third-type area is low, and an amplitude of electrocardiogram data in the fourth-type area that transitions from the first-type area to the third-type area gradually decreases. When smoothing processing is performed on the electrocardiogram data in the fourth-type area by using a plurality of fixed-length windows, if a window length is large, electrocardiogram data near the first-type area may be excessively smoothed, and an electrocardiogram signal feature is lost; or if a window length is small, a feature of electrocardiogram data near the first-type area is retained, but a good smoothing effect is not achieved for electrocardiogram data near the second-type area, and noise may be mistakenly considered as an electrocardiogram feature. Therefore, for the electrocardiogram data in the fourth-type area, it is difficult for the electronic device to find a fixed-length window applicable to the entire fourth-type area.

Based on this, in this embodiment of this application, the window length may be planned based on the amplitude of the electrocardiogram data in the fourth-type area. For the fourth-type area that transitions from the first-type area to the third-type area, the amplitude of electrocardiogram data in the area gradually decreases over time, and correspondingly, a selected window length may gradually increase over time.

In another possible implementation method, when the fourth-type area is an area that transitions from the third-type area to the first-type area, lengths of the plurality of non-fixed-length windows gradually decrease over time. For example, a window length a at a first moment is greater than a window length b at a second moment, and the window length b at the second moment is greater than a window length c at a third moment. By analogy, the window length gradually decreases over time.

It may be understood that an amplitude of electrocardiogram data in the fourth-type area that transitions from the third-type area to the first-type area gradually increases over time, and a window whose length gradually decreases over time may be selected to perform smoothing processing on the electrocardiogram data in the area.

S506. The electronic device integrates the second data, the third data, the fourth data, and the data points in the first-type area, to obtain fifth data obtained after the electrocardiogram data is smoothed.

The electronic device integrates the processed data points in the second-type area, the processed data points in the third-type area, the processed data points in the fourth-type area, and unprocessed data points in the first-type area, to obtain smoothed electrocardiogram data.

In a possible manner, the electronic device updates the data points in the second-type area to data points in the second data, updates the data points in the third-type area to data points in the third data, and updates the data points in the fourth-type area to data points in the fourth data; and the electronic device retains the data points in the first-type area, to update the first data to the fifth data.

In this embodiment of this application, the electronic device preprocesses the electrocardiogram data to obtain the first data. The electronic device divides the data points in the first data into the data points in the first-type area, the data points in the second-type area, and the data points in the buffer-type area. The electronic device performs smoothing processing on the data points in the second-type area by using the plurality of fixed-length windows, to obtain the second data. The electronic device performs smoothing processing on the data points in the third-type area by using the plurality of fixed-length windows, to obtain the third data. The electronic device performs smoothing processing on the data points in the fourth-type area by using the plurality of non-fixed-length windows, to obtain the fourth data. The electronic device integrates the second data, the third data, the fourth data, and the data points in the first-type area, to obtain the fifth data obtained after the electrocardiogram data is smoothed. In this way, the electronic device separately performs smoothing processing on the data points in the second-type area, the third-type area, and the fourth-type area by using different windows, thereby performing smoothing processing on noise in the electrocardiogram signal, and reducing interference of noise to the electrocardiogram signal.

The following describes step S502 in this embodiment of this application in detail with reference to steps S601-S606. For example, that the electronic device divides data points in the first data into data points in a first-type area, data points in a second-type area, data points in a third-type area, and data points in a fourth-type area includes:

The electronic device calculates first variability of the data point in the first data; and the electronic device determines, based on the variability of the data point in the first data and a variability threshold, an area to which the data point in the first data belongs. The variability may reflect a relative degree of discreteness of the data point in the first data, and the electronic device may determine an area type of the data point based on the variability of the data point in the first data.

For example, this embodiment of this application provides a method for distinguishing between the data points in the first-type area, the second-type area, the third-type area, and the fourth-type area based on the variability.

S601. The electronic device calculates first variability of the data point in the first data. The variability includes the first variability and second variability, and the first variability is related to determining the data point in the first-type area, the data point in the third-type area, and the data point in the fourth-type area.

In a possible implementation, the electronic device may select a window whose length is 2k+2 to calculate the first variability $M_1$ of the data point in the first data. That the electronic device calculates the first variability of the data point in the first data may meet the following formula:

$$M_1=\text{mean}(\text{abs}(\text{diff}(X_1)))/(2k+1) \text{ (k is a positive integer)} \quad (1)$$

where $X_1=[x_{t-k-1}, ..., x_t, ...x_{t+k}]$, $X_1$ may be understood as that 2k+2 adjacent data points are extracted from the first data by using the window whose length is 2k+2, xt is a data point in the first data at a moment t, $x_{t-k-1}$ is a data point in the first data at a moment t-k-1, and $x_{t+k}$ is a data point in the first data at a moment t+k. The electronic device may calculate the first variability of the data point xt by using the 2k+2 data points in $X_1$.

Calculation logic of Formula (1) is as follows: For the first variability $M_1$ of the data point xt, the electronic device selects k+1 data points before the data point xt, the data point xt, and k data points after the data point xt as $X_1$. First, the electronic device performs differential processing on the 2k+2 data points in $X_1$, for example, $x_t-x_{t-1}$, to obtain differences between 2k+1 adjacent data points. Then, the electronic device sums and averages absolute values of the differences between the 2k+1 adjacent data points, to obtain the first variability $M_1$ through calculation.

For example, k=2, and $X_1=[1, 3, 4, 2, 3, 2]$, to describe a calculation process of $M_1$.

$$\text{diff}(X_1)=[2, 1, -2, 1, -1]$$

$$\text{abs}(\text{diff}(X_1))= [|2|, |1|, |-2|, |1|, |-1|]=[2, 1, 2, 1, 1]$$

$$\text{mean(abs(diff}(X_1)))=2+1+2+1+1=7$$

$$M_1=\text{mean(abs(diff}(X_1)))/(2k+1)=1.4$$

It may be understood that the foregoing embodiment describes, by using an example, a process in which the electronic device calculates $M_1$ corresponding to a data point xt in the first data. The electronic device may traverse all data points in the first data by using the window whose length is 2k+2, to obtain the first variability $M_1$ corresponding to each data point. A step of traversing another data point is similar to step S601, and details are not described in this embodiment of this application.

S602. When the first variability is less than a first variability threshold, the electronic device determines that the data point in the first data is the data point in the second-type area.

The first variability threshold is used to obtain the data points in the first-type area, the third-type area, and the fourth-type area. The first variability threshold may be $C_1$ (which is obtained based on data statistical experience). In a possible implementation, the first variability threshold may be obtained based on a variability feature curve of historical electrocardiogram data. For example, the electronic device may obtain data points in the historical electrocardiogram data. The electronic device calculates variability of the data point in the historical electrocardiogram data, and defines variability of a boundary point that can be used to distinguish between the second-type area and another-type area (the first-type area, the third-type area, and the fourth-type area) as $C_1$.

The electronic device may calculate $M_1$ of the data point in the first data. When a value of $M_1$ is less than $C_1$, it indicates that the data point is located in the second-type area.

Alternatively, S603. When the first variability is not less than the first variability threshold, the electronic device determines that the data point in the first data is the data point in the first-type area, the data point in the third-type area, or the data point in the fourth-type area.

When the value of $M_1$ is not less than $C_1$, it indicates that the data point is not located in the second-type area, and then the data point may be the data point in the first-type area, the third-type area, or the fourth-type area.

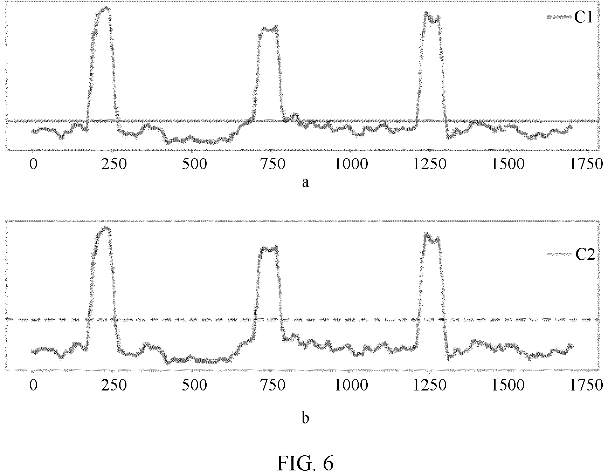

For example, as shown in a in FIG. 6, a in FIG. 6 may be a feature curve of the first variability $M_1$ of the data point in the first data, and a solid line $C_1$ in a in FIG. 6 may be a boundary between the second-type area and another-type area. A data point that is in the first data and whose $M_1$ is below $C_1$ is the data point in the second-type area, and a data point that is in the first data and whose $M_1$ is above $C_1$ may be the data point in the another-type area.

It may be understood that there is no sequence between step S602 and step S603, step S602 is a possible implementation after the electronic device performs step S601, and step S603 is another possible implementation after the electronic device performs step S601. After obtaining a data point in the first data at a moment t, the electronic device calculates the first variability of the data point. If the first variability is less than the first variability threshold, the data point is the data point in the second-type area. The electronic device may no longer divide the data point. If the first variability is not less than the first variability threshold, the data point is not the data point in the second-type area, and the electronic device further needs to continue to determine an area to which the data point belongs. After step S603, the electronic device may further perform step S604.

For example, S604. The electronic device calculates second variability of the data point in the first-type area, the data point in the third-type area, or the data point in the fourth-type area.

The electronic device continues to calculate the data point outside the second-type area, to obtain the second variability. The second variability is related to determining the data point in the first-type area.

In a possible implementation, the electronic device may select a window whose length is 2j+2 to calculate the second variability $M_2$ of the data point in the first data. That the electronic device calculates the second variability of the data point in the first-type area, the third-type area, or the fourth-type area may meet the following formula:

$$M_2=\text{mean(abs(diff}(X_2)))/(2j+1) \text{ (j is a positive integer)} \tag{2}$$

where $X_2=[x_{t-j-1}, ..., x_t, ...x_{t+j}]$, and $X_2$ may be understood as that 2j+2 adjacent data points are extracted from the data by using the window whose length is 2j+2. The electronic device may calculate the second variability of the data point xt by using the 2j+2 data points in $X_2$.

It may be understood that $M_2$ may be obtained by calculating the data point in the first-type area, the third-type area, or the fourth-type area, a quantity of data points needs to be less than a quantity of data points in the first data, and $M_2$ is used to distinguish the first-type area. Therefore, a window for calculating $M_2$ is generally different from a window for calculating $M_1$, that is, j may not be equal to k.

In this embodiment of this application, operation logic of Formula (2) is similar to operation logic of Formula (1), and is not described herein by using an example.

S605. When the second variability is not less than a second variability threshold, the electronic device determines that the data point in the first data is the data point in the first-type area.

The second variability threshold is used to obtain the data point in the first-type area. The second variability threshold may be $C_2$ (which is obtained based on data statistical experience). In a possible implementation, the second variability threshold may be obtained based on a variability feature curve of historical electrocardiogram data. For example, the electronic device may obtain data points in the historical electrocardiogram data. The electronic device calculates variability of the data point in the historical electrocardiogram data, and defines variability of a boundary point that can be used to distinguish between the first-type area and the buffer-type area (the third-type area and the fourth-type area) as $C_2$.

The electronic device may calculate $M_2$ of the data point in the first-type area, the third-type area, or the fourth-type area in step S603. When a value of $M_2$ is not less than $C_2$, it indicates that the data point is located in the first-type area.

Alternatively, S606. When the second variability is less than the second variability threshold, the electronic device determines that the data point in the first data is the data point in the third-type area or the data point in the fourth-type area.

When the value of $M_2$ is less than $C_2$, it indicates that the data point is located in the third-type area or the fourth-type area. For example, as shown in b in FIG. 6, b in FIG. 6 may be a feature curve of a data point $M_2$ in the first-type area, the third-type area, and the fourth-type area in step S604, and a dashed line $C_2$ in b in FIG. 6 may be a boundary between the first-type area and the buffer-type area (the third-type area and the fourth-type area). A data point whose $M_2$ is below $C_2$ is the data point in the third-type area or the fourth-type area, and a data point whose $M_2$ is above $C_2$ may be the data point in the first-type area.

It should be noted that, to facilitate description of the area division method in this embodiment of this application, the feature curve of $M_1$ in step S601 and the feature curve of $M_2$ in step S604 are shown by using an example. In actual operation, the feature curves of both are different, and there is no specific magnitude relationship between $C_1$ and $C_2$.

It may be understood that there is no sequence between step S605 and step S606, step S605 is a possible implementation after the electronic device performs step S604, and step S606 is another possible implementation after the electronic device performs step S604. In this embodiment of this application, the electronic device may perform steps S601-S606 for one data point in the first data to determine an area type to which the data point belongs. Then, the electronic device traverses all data points in the electrocardiogram data by using the method provided in this embodiment of this application, to obtain electrocardiogram data divided into different types of areas, as shown in FIG. 4.

To improve a suppression effect, on electrocardiogram noise, of the electrocardiogram signal processing method in this embodiment of this application, in this embodiment of this application, the data points in the third-type area and the data points in the fourth-type areas are further divided. The following describes division methods of the third-type area and the fourth-type area.

In addition to the first-type area and the second-type area, there is a buffer-type area in the first data, and the buffer-type area may include the third-type area and the fourth-type area. In a possible implementation, in this embodiment of this application, an area with a preset length in the buffer-type area may be selected at a boundary between the second-type area and the buffer-type area as the data points in the third-type area. The third-type area is an area, with a preset length, that is in the buffer-type area and that is adjacent to a boundary of the second-type area.

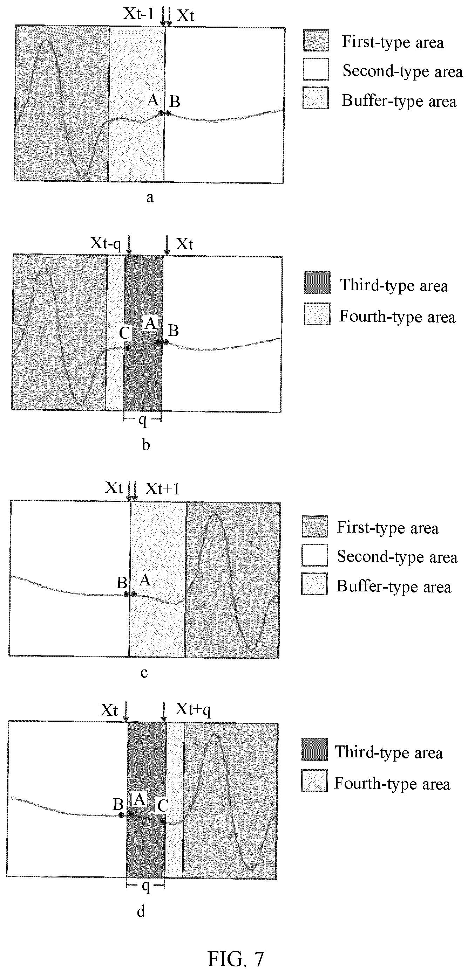

For example, after performing steps S601-S606, the electronic device may obtain the data points in the first-type area, the data points in the second-type area, and the data points in the buffer-type area. As shown in a in FIG. 7, a data point $x_{t-1}$ is located at a point A, and a data point xt is located at a point B, where the data point xt and the data point $x_{t-1}$ are two adjacent data points, the data point $x_{t-1}$ is located in the buffer-type area, and the data point xt is located in the second-type area. In this case, the boundary between the second-type area and the buffer-type area may exist between the data point $x_{t-1}$ and the data point $x_t$.

The area with the preset length may be the third-type area shown in b in FIG. 7, and the preset length q may be customized. Starting from the boundary, the electronic device selects an area whose length is q from the buffer-type area, to obtain the third-type area. The data points in the third-type area may include $[x_{t-q}, ..., x_t)$.

For another example, as shown in c and d in FIG. 7, a data point xt is located in the second-type area, a data point $x_{t+1}$ is located in the buffer-type area, and the data point xt and the data point $x_{t+1}$ are two adjacent data points. In this case, the boundary between the buffer-type area and the second-type area may exist between the data point $x_{t+1}$ and the data point xt. Starting from the boundary, the electronic device selects an area whose length is q from the buffer-type area, to obtain the third-type area. The data points in the third-type area may include $(xt, ..., x_{t+q}]$. It may be understood that the electrocardiogram signal may be considered as a periodic signal, and two buffer-type areas may be included in a single period. Methods for processing the data points in the buffer-type area by the electronic device are similar. In this embodiment of this application, c and d in FIG. 7 are not described in detail.

It may be understood that, a remaining area other than the third-type area in the buffer-type area is the fourth-type area. The buffer-type area is a transition area between the first-type area and the second-type area, and the buffer-type area may include an area with a high amplitude and an area with a low amplitude. In this embodiment of this application, this area may be further divided into the third-type area and the fourth-type area, to improve an effect of subsequent smoothing processing on the electrocardiogram signal.

The foregoing embodiment provides an area division method for electrocardiogram data. The following describes a method for performing smoothing processing on data points in various areas in this embodiment of this application.

S701. The electronic device does not process the data points in the first-type area.

After performing step S605, the electronic device may obtain the data points in the first-type area. In this case, the electronic device may perform step S701. An amplitude of the data point in the first-type area is high, and if the electronic device performs smoothing processing on the data point in the area, a signal feature of the data point in the area may be lost. Therefore, for the data point in the area, the electronic device may directly output the data point in the first-type area without performing smoothing the data point, that is, $x_t^{r_1} = x_t$.

S702. The electronic device performs smoothing processing on the data points in the second-type area by using a plurality of fixed-length windows, to obtain second data.

After performing step S602, the electronic device may obtain the data points in the second-type area. In this case, the electronic device may perform step S702. An amplitude of the data point in the second-type area is low and smooth, and a range covered the second-type area is large. For the data points in the area, the electronic device may perform smoothing processing on the data points in the second-type area by using the fixed-length windows, to obtain the second data.

A data point $x_t^{r_2}$ in the second data may meet the following formula:

$$x_t^{r_2} = (x_{t-H} + \ldots + x_t +, \ldots, + x_{t+H})/(2H +$$

$$1) \text{ (H is a positive integer and H is a constant)} \tag{3}$$

where a length of a window $X_3$ is 2H+1, the length of the window $X_3$ is fixed, $X_3 = [x_{t-H}, \ldots, x_t, \ldots, x_{t+H}]$, $x_t$ is a data point in the first data at a moment t, $x_{t-H}$ is a data point in the first data at a moment t-H, and $x_{t+H}$ is a data point in the first data at a moment t+H.

It may be understood that the window $X_3$ may include 2H+1 data points including H data points before the data point xt and H data points after the data point xt. The electronic device sums and then averages the data points in the window $X_3$, to obtain the data point $x_t^{r_2}$ that is in the second data and that corresponds to the data point xt. In this embodiment of this application, the electronic device may implement a noise suppression effect by processing an amplitude of a data point at which noise is located as an average value of amplitudes of adjacent data points around the data point.

S703. The electronic device performs smoothing processing on the data points in the third-type area by using a plurality of fixed-length windows, to obtain third data. A length of a fixed window used to perform smoothing processing on the third-type area is less than a length of a fixed window used to perform smoothing processing on the second-type area.

After performing step S606, the electronic device may obtain the data points in the third-type area. In this case, the electronic device may perform step S703. The third-type area is a part of the buffer-type area, and the third-type area is adjacent to the second-type area. An amplitude change of the data point in the third-type area is smooth, and the electronic device may still perform smoothing processing on the third-type area by using the fixed-length window. However, a range covered by the third-type area is less than the range covered by the second-type area, and an amplitude of the data point in the fourth-type area may be slightly higher. Therefore, the window length used by the electronic device to process the data point in the third-type area may be less than the window length used to process the data point in the second-type area.

A data point $x_t^{r_3}$ in the third data may meet the following formula:

$$x_t^{r_3} = (x_{t-Q} + \ldots + x_t +, \ldots, + x_{t+Q})/(2Q +$$

$$1)(Q \text{ is a positive integer and Q is a constant, and } Q < H) \tag{4}$$

where a length of a window $X_4$ is 2Q+1, the length of the window $X_4$ is fixed, $X_4 = [x_{t-Q}, \ldots, x_t, \ldots, x_{t+Q}]$, xt is a data point in the first data at a moment t, $x_{t-Q}$ is a data point in the first data at a moment t-Q, and $x_{t+Q}$ is a data point in the first data at a moment t+Q. It may be understood that the third-type area is the area with the preset length q. When calculating the data point in the third-type area, the electronic device selects a window length that should not exceed the length of the third-type area. Therefore, a value range of the length of the window $X_4$ is [1, q].

S704. The electronic device performs smoothing processing on the data points in the fourth-type area by using a plurality of non-fixed-length windows, to obtain fourth data.

After performing step S606, the electronic device may obtain the data points in the fourth-type area. In this case, the electronic device may perform step S704. The fourth-type area is a part of the buffer-type area, the fourth-type area is adjacent to the first-type area, and an amplitude difference between adjacent data points in the fourth-type area may reflect a gradual increase or decrease trend. For example, when the data points in the fourth-type area are close to the first-type area, the amplitude difference between adjacent data points is large. When the data points in the fourth-type area are close to the third-type area, the amplitude difference between adjacent data points is small. For characteristics of the data points in the fourth-type area, the electronic device may perform smoothing processing on the data points in the fourth-type area by using a plurality of non-fixed-length windows, to obtain the fourth data.

$$x_t^{r_4} = (x_{t-u}+\ldots+x_t+,\ldots,+x_{t+u})/(2u +$$

$$1)(u \text{ is a positive integer and } u \text{ is a variable}) \tag{5}$$

where a length of a window $X_5$ is 2u+1, the length of the window $X_5$ is variable, $X_5=[x_{t-u}, \ldots, x_t, \ldots, x_{t+u}]$, xt is a data point in the first data at a moment t, $x_{t-u}$ is a data point in the first data at a moment t-u, and $x_{t+u}$ is a data point in the first data at a moment t+u.

For example, one period of the electrocardiogram data may include two fourth-type areas. The fourth-type area may be an area that transitions from the first-type area to the third-type area, or the fourth-type area may be an area that transitions from the third-type area to the first-type area.

In a possible implementation, when the fourth-type area is an area that transitions from the first-type area to the third-type area, an amplitude of the data point in the fourth-type area gradually decreases. Therefore, for a data point that is in the fourth-type area and that is close to the first-type area, the electronic device may select a window with a short length to perform smoothing processing. For a data point that is in the fourth-type area and that is close to the third-type area, the electronic device may select a long window to perform smoothing processing. A length of the window $X_5$ gradually increases, and the plurality of non-fixed-length windows may be a plurality of windows whose lengths gradually increase over time. For example, a value of u may be [1, 2, ..., Q).

In another possible implementation, when the fourth-type area is an area that transitions from the third-type area to the first-type area, an amplitude of the data point in the fourth-type area gradually increases. Therefore, for a data point that is in the fourth-type area and that is close to the first-type area, the electronic device may select a short window to perform smoothing processing. For a data point that is in the fourth-type area and that is close to the third-type area, the electronic device may select a long window to perform smoothing processing. A length of the window $X_5$ gradually decreases, and the plurality of non-fixed-length windows may be a plurality of windows whose lengths gradually decrease over time. For example, a value of u may be (Q, ..., 2, 1].

It should be noted that there is no sequence between steps S701-S704. After obtaining the data point xt in the first data, the electronic device may determine, based on steps S601-S606, an area to which the data point belongs. Correspondingly, the electronic device selects a corresponding area processing method based on the area of the data point.

S705. The electronic device integrates the second data, the third data, the fourth data, and the data points in the first-type area, to obtain fifth data obtained after the electrocardiogram data is smoothed.

The electronic device may integrate the data obtained in steps S701-S704. For example, the electronic device reconstructs the data point $x_t^{r_1}$, the data point $x_t^{r_2}$, the data point $x_t^{r_3}$, and the data point $x_t^{r_4}$, and outputs the fifth data obtained after the electrocardiogram data is smoothed.

In this embodiment of this application, the electronic device performs classification processing on the data points in the first data, and outputs the fifth data obtained after smoothing. In this way, a suppression effect on noise in the electrocardiogram data can be improved, and impact of irregular noise in the electrocardiogram data on the electrocardiogram signal can be reduced.

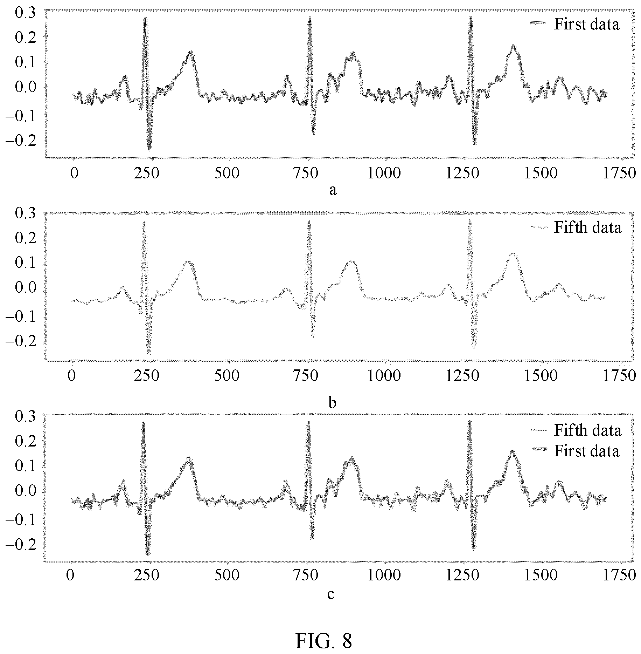

An application scenario of the electrocardiogram signal processing method provided in this embodiment of this application is described below with reference to FIG. 8. As shown in FIG. 8:

When the electronic device receives a trigger operation used to start electrocardiogram signal detection, the electronic device displays data points in the fifth data.

For example, the electronic device receives the trigger operation used to start electrocardiogram signal detection. In response to the trigger operation used to start electrocardiogram signal detection, the electronic device starts to collect electrocardiogram data. After collecting each data point in the electrocardiogram data, the electronic device preprocesses the data point to obtain the data point in the first data. The electronic device processes the data point in the first data into the data point in the fifth data, and displays the data point in the fifth data on a display screen. After traversing all data points, the electronic device may display the fifth data.

It may be understood that, in this embodiment of this application, after collecting the electrocardiogram data, the electronic device may process and display the fifth data obtained after smoothing processing. In some embodiments, the electronic device may delay displaying the fifth data. For example, based on a window, the electronic device obtains, through division, an area to which the data point in the first data belongs and performs smoothing processing. In this case, when calculating a data point, the electronic device needs to ensure that n data points still exist after the data point, and a value of n is not less than a maximum value among k, j, H, Q, and u, that is, $n \geq max(k, j, H, Q, u)$. When collecting a data point $x_{t+n}$, the electronic device may output a data point $x_t^{r_i}$ in the fifth data on the display screen. However, in an actual operation process, time consumed by the electronic device to collect the n data points is very short. This may be considered that the electronic device displays the fifth data in real time.

A curve diagram of the electrocardiogram signal may be shown in FIG. 8, a in FIG. 8 is a curve diagram of the first data, and b in FIG. 8 is a curve diagram of the fifth data. It may be learned that, when the amplitude of the data point in the first data is low, the electrocardiogram signal is greatly affected by noise, and an unsmooth curve appears. For the curve of the fifth data processed by using the method in this embodiment of this application, the curve is relatively smooth in a waveband with a low amplitude. c in FIG. 8 is a comparison diagram of the first data and the fifth data. For the first-type area, the fifth data overlaps the first data, so that an electrocardiogram signal feature of the data point in the first-type area can be fully retained. For another area, the fifth data is smoother than the first data. This indicates that the electrocardiogram signal processing method provided in this embodiment of this application can be used to effectively suppress impact of noise on the electrocardiogram signal. The curve diagram shown in b in FIG. 8 may be displayed on the display screen of the electronic device.

In a possible implementation, the electronic device may store the original electrocardiogram data, the first data, and the fifth data. In another possible implementation, after obtaining the first data based on the original electrocardiogram data, the electronic device may delete the original electrocardiogram data. After obtaining the fifth data through processing based on the first data, the electronic device deletes the first data, so that memory occupied by the data can be reduced. This is not limited in this embodiment of this application.

The electrocardiogram signal processing method provided in the embodiments of this application is described above with reference to FIG. 1-FIG. 8, and an apparatus that is provided in the embodiments of this application and that is configured to perform the foregoing method is described below.

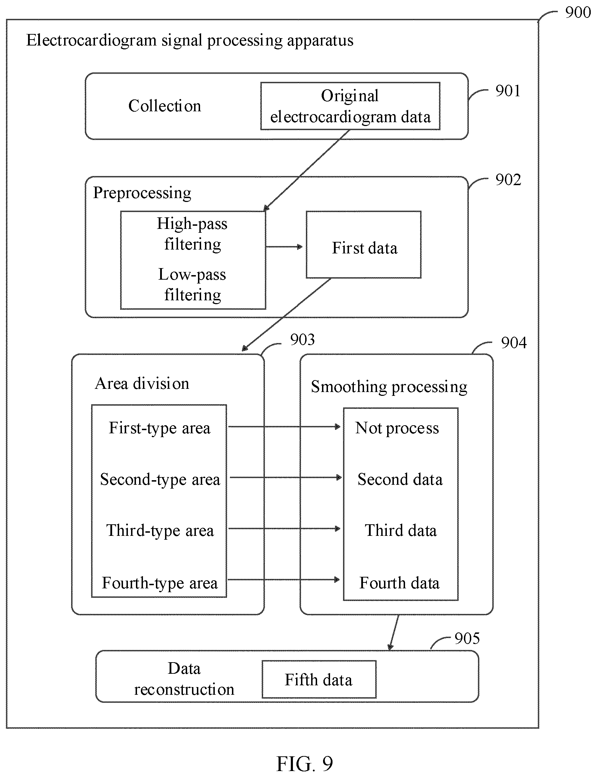

For example, FIG. 9 shows an electrocardiogram signal processing apparatus 900 according to an embodiment of this application. The apparatus includes a collection module 901, a preprocessing module 902, an area division module 903, a smoothing processing module 904, and a data reconstruction module 905.

The collection module 901 is configured to collect original electrocardiogram data. For example, the electrocardiogram data may be electrocardiography (Electrocardiography, ECG) data.

The preprocessing module 902 is configured to perform steps related to preprocessing in this embodiment of this application. For example, the preprocessing module may perform step S501 to perform conventional noise reduction processing on the electrocardiogram data through high-pass filtering and low-pass filtering, to obtain first data.

The area division module 903 is configured to divide the first data into a first-type area, a second-type area, a third-type area, and a fourth-type area. For example, the area division module may perform steps S601-S606, so that the electronic device obtains data points in the first-type area, data points in the second-type area, data points in the third-type area, and data points in the fourth-type area.

The smoothing processing module 904 is configured to perform smoothing processing on data points in different types of areas by using windows with different lengths, to separately obtain second data, third data, and fourth data. For example, the smoothing processing module may perform steps S701-S704 to obtain data obtained after smoothing processing.

The data reconstruction module 905 is configured to integrate the second data, the third data, the fourth data, and the data points in the first-type area, to obtain fifth data obtained after the electrocardiogram data is smoothed. For example, the data reconstruction module may perform step S705 to obtain and output the fifth data.

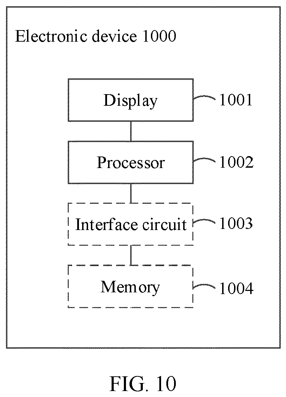

An electronic device that is provided in the embodiments of this application and that is configured to perform the foregoing method is described below. FIG. 10 is a schematic diagram of a structure of an electronic device that supports the electrocardiogram signal processing method according to an embodiment of this application. The electronic device may be a terminal device in the embodiments of this application, or may be a chip or a chip system in the terminal device.

As shown in FIG. 10, the electronic device 1000 may be used in a communication device, a circuit, a hardware component, or a chip. The electronic device includes a display 1001 and a processor 1002. The display 1001 is configured to support the electronic device in performing a display step. The processor 1002 is configured to support the electronic device in performing an information processing step.

In a possible implementation, the electronic device 1000 may also include an interface circuit 1003. Specifically, the interface circuit is configured to support the electronic device 1000 in performing steps of data sending and data receiving. The interface circuit 1003 may be an input or output interface, a pin, a circuit, or the like.

In a possible embodiment, the electronic device may further include a memory 1004. The processor 1002 and the memory 1004 are connected to each other by using a line. The memory 1004 may be one or more components that are in a device or a circuit and that are configured to store a program or data. The memory 1004 may exist independently, and is connected to the processor 1002 of the electronic device by using the communication line. Alternatively, the memory 1004 may be integrated with the processor 1002.

The memory 1004 may store computer-executable instructions of the method in the terminal device, so that the processor 1002 performs the method in the foregoing embodiments. The memory 1004 may be a register, a cache, a RAM, or the like, and the memory 1004 may be integrated with the processor 1002. The memory 1004 may be a read-only memory (read-only memory, ROM) or another type of static storage device that may store static information and instructions. The memory 1004 may be independent of the processor 1002.

In the foregoing embodiments, the instructions stored in the memory for execution by the processor may be implemented in a form of a computer program product. The computer program product may be written in the memory in advance, or may be downloaded and installed in the memory in a form of software.

The computer program product includes one or more computer instructions. When the computer program instructions are loaded and executed on a computer, all or some of the procedures or functions according to embodiments of this application are generated. The computer may be a general-purpose computer, a special-purpose computer, a computer network, or another programmable apparatus. The computer instructions may be stored in a computer-readable storage medium or transmitted from one computer-readable storage medium to another computer-readable storage medium. For example, the computer instructions may be transmitted from one website, computer, server, or data center to another website, computer, server, or data center in a wired (for example, a coaxial cable, an optical fiber, or a digital subscriber line (digital subscriber line, DSL)) manner or a wireless (for example, infrared, radio, or microwave) manner. The computer-readable storage medium may be any available medium accessible by the computer, or a data storage device such as a server or a data center integrating one or more available media. For example, the available medium may include a magnetic medium (for example, a floppy disk, a hard disk, or a magnetic tape), an optical medium (for example, a digital versatile disc (digital versatile disc, DVD)), a semiconductor medium (for example, a solid state disk (solid state disk, SSD)), or the like.

An embodiment of this application further provides a computer-readable storage medium. All or some of the methods described in the foregoing embodiments may be implemented by using software, hardware, firmware, or any combination thereof. The computer-readable medium may include a computer storage medium and a communication medium, and may further include any medium that enables a computer program to be transmitted from one place to another place. The storage medium may be any target medium accessible by the computer.

In a possible design, the computer-readable medium may include a compact disc read-only memory (compact disc read-only memory, CD-ROM), a RAM, a ROM, an EEPROM, or another optical disc memory; or the computer-readable medium may include a magnetic disk memory or another magnetic disk storage device. In addition, any connection line may also be appropriately referred to as a computer-readable medium. For example, if software is transmitted from a website, a server, or another remote source by using a coaxial cable, an optical fiber cable, a twisted pair, a DSL, or wireless technologies (for example, infrared, radio, and microwave), the coaxial cable, the optical fiber cable, the twisted pair, the DSL, or the wireless technologies such as infrared, radio, and microwave are included in the definition of the medium. As used herein, a magnetic disk and an optical disc include a compact disc (CD), a laser disc, an optical disc, a digital versatile disc (digital versatile disc, DVD), a floppy disk, and a Blu-ray disc, and the magnetic disk usually reproduces data magnetically, while the optical disc reproduces data optically by using a laser.

The foregoing combinations should also be included in the scope of the computer-readable medium. The foregoing descriptions are merely specific implementations of the present invention. However, the protection scope of the present invention is not limited thereto. Therefore, the protection scope of the present invention shall be subject to the protection scope of the claims.

**Claims**

1. An electrocardiogram signal processing method, applied to an electronic device, wherein the method comprises:

preprocessing, by the electronic device, electrocardiogram data to obtain first data, wherein the preprocessing is used to filter out baseline drift and power frequency interference noise in the electrocardiogram data;
dividing, by the electronic device, data points in the first data into data points in a first-type area, data points in a second-type area, and data points in a buffer-type area, wherein the first-type area is a part of a QRS waveband in the electrocardiogram data, the first-type area comprises an R wave, the second-type area comprises an area from a start point of a T wave to an end point of a P wave, the buffer-type area comprises a third-type area and a fourth-type area, the third-type area is an area, with a preset length, that is in the buffer-type area and that is adjacent to a boundary of the second-type area, and the fourth-type area is an area other than the third-type area in the buffer-type area;

performing, by the electronic device, smoothing processing on the data points in the second-type area by using a plurality of fixed-length windows, to obtain second data;

performing, by the electronic device, smoothing processing on the data points in the third-type area by using a plurality of fixed-length windows, to obtain third data, wherein a length of a fixed window used to perform smoothing processing on the third-type area is less than a length of a fixed window used to perform smoothing processing on the second-type area;

performing, by the electronic device, smoothing processing on the data points in the fourth-type area by using a plurality of non-fixed-length windows, to obtain fourth data; and

integrating, by the electronic device, the second data, the third data, the fourth data, and the data points in the first-type area, to obtain fifth data obtained after the electrocardiogram data is smoothed.

2. The method according to claim 1, wherein when the fourth-type area is an area that transitions from the first-type area to the third-type area, lengths of the plurality of non-fixed-length windows gradually increase over time; or when the fourth-type area is an area that transitions from the third-type area to the first-type area, lengths of the plurality of non-fixed-length windows gradually decrease over time.

3. The method according to claim 1 or 2, wherein first variability is related to determining the data point in the first-type area, the data point in the third-type area, and the data point in the fourth-type area, and the dividing, by the electronic device, data points in the first data into data points in a first-type area, data points in a second-type area, data points in a third-type area, and data points in a fourth-type area comprises:

calculating, by the electronic device, the first variability of the data point in the first data; and when the first variability is less than a first variability threshold, determining, by the electronic device, that the data point in the first data is the data point in the second-type area; or when the first variability is not less than the first variability threshold, determining, by the electronic device, that the data point in the first data is the data point in the first-type area, the data point in the third-type area, or the data point in the fourth-type area.

4. The method according to claim 3, wherein second variability is related to determining the data point in the first-type area, and the dividing, by the electronic device, data points in the first data into data points in a first-type area, data points in a second-type area, data points in a third-type area, and data points in a fourth-type area further comprises:

calculating, by the electronic device, the second variability of the data point in the first-type area, the data point in the third-type area, or the data point in the fourth-type area; and when the second variability is not less than a second variability threshold, determining, by the electronic device, that the data point in the first data is the data point in the first-type area; or when the second variability is less than the second variability threshold, determining, by the electronic device, that the data point in the first data is the data point in the third-type area or the data point in the fourth-type area.

5. The method according to claim 4, wherein the first variability $M_1$ meets the following formula:

$M_1$=mean(abs(diff($X_1$)))/(2k+1), wherein k is a positive integer, a window length selected when $M_1$ is calculated is 2k+2, and $X_1$ is a set of 2k+2 data points in the first data in a window; and the second variability $M_2$ meets the following formula:
$M_2$=mean(abs(diff($X_2$)))/(2j+1), wherein j is a positive integer, a window selected when $M_2$ is calculated is 2j+2, and $X_2$ is a set of 2j+2 data points in the first data in the window.

6. The method according to any one of claims 1-5, wherein $x_t^{r_2}$ is a data point in the second data at a moment t, and the data point $x_t^{r_2}$ in the second data meets the following formula:

$$x_t^{r_2} = (x_{t-H} + \ldots + x_t + , \ldots, + x_{t+H})/(2H + 1),$$

wherein xt is a data point in the first data at the moment t, $x_{t-H}$ is a data point in the first data at a moment t-H, $x_{t+H}$ is a data point in the first data at a moment t+H, H is a positive integer and H is a constant, and a window length selected when $x_t^{r_2}$ is calculated is 2H+1.

7. The method according to any one of claims 1-5, wherein the preset length is q, $x_t^{r_3}$ is a data point in the third data at a moment t, and the data point $x_t^{r_3}$ in the third data meets the following formula:

$$x_t^{r_3} = (x_{t-Q} + \ldots + x_t + , \ldots, + x_{t+Q})/(2Q + 1),$$

wherein $x_{t-Q}$ is a data point in the first data at a moment t-Q, $x_{t+Q}$ is a data point in the first data at a moment t+Q, Q is a positive integer and Q is a constant, Q<H, a window length selected when $x_t^{r_3}$ is calculated is 2Q+1, and a value range of the window length is [1, q].

8. The method according to any one of claims 1-5, wherein $x_t^{r_4}$ is a data point in the fourth data at a moment t, and the data point $x_t^{r_4}$ in the fourth data meets the following formula:

$$x_t^{r_4} = (x_{t-u} + \ldots + x_t + , \ldots, + x_{t+u})/(2u + 1),$$

wherein $x_{t-u}$ is a data point in the first data at a moment t-u, $x_{t+u}$ is a data point in the first data at a moment t+u, u is a positive integer and u is a variable, and a window length selected when $x_t^{r_4}$ is calculated is 2u+1.

9. The method according to any one of claims 1-8, wherein the method further comprises:
when the electronic device receives a trigger operation used to start electrocardiogram data detection, displaying, by the electronic device, data points in the fifth data.

10. The method according to any one of claims 1-9, wherein the electronic device comprises a wearable device, and the wearable device comprises one or more of the following: a smartwatch, a smart band, or smart glasses.

11. An electronic device, comprising a memory, a processor, and a computer program stored in the memory and capable of running on the processor, wherein when the processor executes the computer program, the electronic device is enabled to perform the method according to any one of claims 1-10.

12. A computer-readable storage medium, wherein the computer-readable storage medium stores a computer program, and when the computer program is executed by a processor, a computer is enabled to perform the method according to any one of claims 1-10.

13. A computer program product, comprising a computer program, wherein when the computer program is run, a computer is enabled to perform the method according to any one of claims 1-10.

**Patentansprüche**

1. Ein Verfahren zur Signalverarbeitung eines Elektrokardiogramms, angewendet auf ein elektronisches Gerät, wobei das Verfahren Folgendes umfasst:

Vorverarbeiten der Elektrokardiogramm-Daten durch das elektronische Gerät, um erste Daten zu erhalten, wobei die Vorverarbeitung dazu dient, Basisliniendrift und Störgeräusche der Netzfrequenz in den Elektrokardiogramm-Daten herauszufiltern;

Unterteilen der Datenpunkte in den ersten Daten durch das elektronische Gerät in Datenpunkte eines Bereichs ersten Typs, Datenpunkte eines Bereichs zweiten Typs und Datenpunkte eines Bereichs des Puffertyps, wobei der Bereich ersten Typs ein Teil eines QRS-Bands der Elektrokardiogramm-Daten ist, der Bereich ersten Typs eine R-Welle umfasst, der Bereich zweiten Typs einen Bereich von einem Anfangspunkt einer T-Welle bis zu einem Endpunkt einer P-Welle umfasst, der Bereich des Puffertyps einen Bereich dritten Typs und einen Bereich vierten Typs umfasst, der Bereich dritten Typs ein Bereich mit einer voreingestellten Länge ist, der sich im Bereich des Puffertyps befindet und an eine Grenze des Bereichs zweiten Typs angrenzt, und der Bereich vierten Typs ein Bereich im Bereich des Puffertyps außer dem Bereich dritten Typs ist;

Durchführen einer Glättungsverarbeitung der Datenpunkte im Bereich zweiten Typs durch das elektronische Gerät mittels einer Vielzahl von Fenstern fester Länge, um zweite Daten zu erhalten;

Durchführen einer Glättungsverarbeitung der Datenpunkte im Bereich dritten Typs durch das elektronische Gerät mittels einer Vielzahl von Fenstern fester Länge, um dritte Daten zu erhalten, wobei die Länge eines festen Fensters, das zur Glättung des Bereichs dritten Typs verwendet wird, kürzer ist als die Länge eines festen Fensters, das zur Glättung des Bereichs zweiten Typs verwendet wird;

Durchführen einer Glättungsverarbeitung der Datenpunkte im Bereich vierten Typs durch das elektronische Gerät mittels einer Vielzahl von Fenstern variabler Länge, um vierte Daten zu erhalten; und

Integrieren der zweiten, dritten und vierten Daten sowie der Datenpunkte im Bereich ersten Typs durch das elektronische Gerät, um Fünfte Daten zu erhalten, die nach der Glättung der Elektrokardiogramm-Daten erhalten werden.

2. Verfahren nach Anspruch 1, wobei, wenn der Bereich vierten Typs ein Bereich ist, der vom Bereich ersten Typs in den Bereich dritten Typs übergeht, sich die Längen der Vielzahl von Fenstern variabler Länge im Zeitverlauf allmählich vergrößern; oder

wenn der Bereich vierten Typs ein Bereich ist, der vom Bereich dritten Typs in den Bereich ersten Typs übergeht, sich die Längen der Vielzahl von Fenstern variabler Länge im Zeitverlauf allmählich verkleinern.

3. Verfahren nach Anspruch 1 oder 2, wobei die erste Variabilität sich auf die Bestimmung des Datenpunkts im Bereich des ersten Typs, des Datenpunkts im Bereich des dritten Typs und des Datenpunkts im Bereich des vierten Typs bezieht und das Aufteilen der Datenpunkte in den ersten Daten durch die elektronische Vorrichtung in Datenpunkte eines Bereichs des ersten Typs, Datenpunkte eines Bereichs des zweiten Typs, Datenpunkte eines Bereichs des dritten Typs und Datenpunkte eines Bereichs des vierten Typs Folgendes umfasst:

Berechnung der ersten Variabilität des Datenpunkts in den ersten Daten durch die elektronische Vorrichtung; und

Wenn die erste Variabilität geringer als ein erster Variabilitätsschwellenwert ist, bestimmt die elektronische Vorrichtung, dass der Datenpunkt in den ersten Daten der Datenpunkt im Bereich des zweiten Typs ist; oder

Wenn die erste Variabilität nicht kleiner als der erste Variabilitätsschwellenwert ist, bestimmt die elektronische Vorrichtung, dass der Datenpunkt in den ersten Daten der Datenpunkt im Bereich des ersten Typs, des dritten Typs oder des vierten Typs ist.

4. Verfahren nach Anspruch 3, wobei sich die zweite Variabilität auf die Bestimmung des Datenpunkts im Bereich des ersten Typs bezieht und das Aufteilen der Datenpunkte in den ersten Daten durch die elektronische Vorrichtung in Datenpunkte eines Bereichs des ersten Typs, Datenpunkte eines Bereichs des zweiten Typs, Datenpunkte eines Bereichs des dritten Typs und Datenpunkte eines Bereichs des vierten Typs weiterhin Folgendes umfasst:

Berechnung der zweiten Variabilität des Datenpunkts im Bereich des ersten Typs, des Datenpunkts im Bereich des dritten Typs oder des Datenpunkts im Bereich des vierten Typs durch die elektronische Vorrichtung; und

Wenn die zweite Variabilität nicht kleiner als ein zweiter Variabilitätsschwellenwert ist, bestimmt die elektronische Vorrichtung, dass der Datenpunkt in den ersten Daten der Datenpunkt im Bereich des ersten Typs ist; oder

Wenn die zweite Variabilität geringer als der zweite Variabilitätsschwellenwert ist, bestimmt die elektronische Vorrichtung, dass der Datenpunkt in den ersten Daten der Datenpunkt im Bereich des dritten Typs oder des vierten Typs ist.

5. Verfahren nach Anspruch 4, wobei die erste Variabilität M1 die folgende Formel erfüllt:

$M_1=\text{mean}(\text{abs}(\text{diff}(X_1)))/(2k+1)$, wobei k eine positive ganze Zahl ist, eine beim Berechnen von $M_1$ gewählte Fensterlänge $2k+2$ ist, und $X_1$ eine Gruppe von $2k+2$ Datenpunkten in den ersten Daten in einem Fenster ist; und

die zweite Variabilität M2 erfüllt die folgende Formel:
M$_2$=mean(abs(diff(X$_2$)))/(2j+1), wobei j eine positive ganze Zahl ist, ein beim Berechnen von M$_2$ gewähltes Fenster 2j+2 ist, und X$_2$ eine Gruppe von 2j+2 Datenpunkten in den ersten Daten im Fenster ist.

6. Verfahren nach einem der Ansprüche 1-5, wobei $x_t^{r_2}$ ein Datenpunkt in den zweiten Daten zum Zeitpunkt t ist und der Datenpunkt $x_t^{r_2}$ in den zweiten Daten folgende Formel erfüllt:

$$x_t^{r_2} = (x_{t-H} + \ldots + x_t + \ldots, + x_{t+H})/(2H + 1),$$

wobei xt ein Datenpunkt in den ersten Daten zum Zeitpunkt t ist, x$_{t-H}$ ein Datenpunkt in den ersten Daten zum Zeitpunkt t-H ist, x$_{t+H}$ ein Datenpunkt in den ersten Daten zum Zeitpunkt t+H ist, H eine positive ganze Zahl und H eine Konstante ist, und eine bei der Berechnung von $x_t^{r_2}$ ausgewählte Fensterlänge 2H+1 beträgt.

7. Verfahren nach einem der Ansprüche 1-5, wobei die voreingestellte Länge q ist, $x_t^{r_3}$ ein Datenpunkt in den dritten Daten zum Zeitpunkt t ist und der Datenpunkt $x_t^{r_3}$ in den dritten Daten folgende Formel erfüllt:

$$x_t^{r_3} = (x_{t-Q} + \ldots + x_t + \ldots, + x_{t+Q})/(2Q + 1),$$

wobei x$_{t-Q}$ ein Datenpunkt in den ersten Daten zum Zeitpunkt t-Q ist, x$_{t+Q}$ ein Datenpunkt in den ersten Daten zum Zeitpunkt t+Q ist, Q eine positive ganze Zahl und Q eine Konstante ist, Q<H, eine bei der Berechnung von $x_t^{r_3}$ ausgewählte Fensterlänge 2Q+1 beträgt, und der Wertebereich der Fensterlänge [1, q] ist.

8. Verfahren nach einem der Ansprüche 1-5, wobei $x_t^{r_4}$ ein Datenpunkt in den vierten Daten zum Zeitpunkt t ist und der Datenpunkt $x_t^{r_4}$ in den vierten Daten folgende Formel erfüllt:

$$x_t^{r_4} = (x_{t-u} + \ldots + x_t + \ldots, + x_{t+u})/(2u + 1),$$

wobei x$_{t-u}$ ein Datenpunkt in den ersten Daten zum Zeitpunkt t-u ist, x$_{t+u}$ ein Datenpunkt in den ersten Daten zum Zeitpunkt t+u ist, u eine positive ganze Zahl und u eine Variable ist, und eine bei der Berechnung von $x_t^{r_4}$ ausgewählte Fensterlänge 2u+1 beträgt.

9. Verfahren nach einem der Ansprüche 1-8, wobei das Verfahren weiterhin Folgendes umfasst:
Wenn das elektronische Gerät eine Trigger-Operation empfängt, die für den Start der Elektrokardiogramm-Datenerkennung verwendet wird, zeigt das elektronische Gerät Datenpunkte in den fünften Daten an.

10. Verfahren nach einem der Ansprüche 1-9, wobei das elektronische Gerät ein tragbares Gerät umfasst und das tragbare Gerät eines oder mehrere der Folgenden umfasst: eine Smartwatch, ein Smartband oder eine intelligente Brille.

11. Elektronisches Gerät, umfassend einen Speicher, einen Prozessor und ein im Speicher gespeichertes Computerprogramm, das auf dem Prozessor lauffähig ist, wobei beim Ausführen des Computerprogramms durch den Prozessor das elektronische Gerät in die Lage versetzt wird, das Verfahren nach einem der Ansprüche 1-10 auszuführen.

12. Computerlesbares Speichermedium, wobei das computerlesbare Speichermedium ein Computerprogramm speichert und beim Ausführen des Computerprogramms durch einen Prozessor ein Computer in die Lage versetzt wird, das Verfahren nach einem der Ansprüche 1-10 auszuführen.

**13.** Computerprogrammprodukt, umfassend ein Computerprogramm, wobei beim Ausführen des Computerprogramms ein Computer in die Lage versetzt wird, das Verfahren nach einem der Ansprüche 1-10 auszuführen.

**Revendications**

**1.** Procédé de traitement de signal d'électrocardiogramme, appliqué à un dispositif électronique, ledit procédé comprenant :

prétraiter, par le dispositif électronique, les données d'électrocardiogramme pour obtenir des premières données, le prétraitement étant utilisé pour filtrer la dérive de la ligne de base et les bruits d'interférence de la fréquence secteur dans les données d'électrocardiogramme ;
diviser, par le dispositif électronique, les points de données des premières données en points de données dans une zone de premier type, points de données dans une zone de second type et points de données dans une zone tampon, la zone de premier type étant une partie d'une bande QRS dans les données d'électrocardiogramme, la zone de premier type comprenant une onde R, la zone de second type comprenant une zone allant d'un point de départ d'une onde T à un point final d'une onde P, la zone tampon comprenant une zone de troisième type et une zone de quatrième type, la zone de troisième type étant une zone, de longueur prédéfinie, qui se trouve dans la zone tampon et est adjacente à la limite de la zone de second type, et la zone de quatrième type étant une zone autre que la zone de troisième type dans la zone tampon ;
effectuer, par le dispositif électronique, un lissage sur les points de données dans la zone de second type en utilisant une pluralité de fenêtres de longueur fixe, pour obtenir des deuxièmes données ;
effectuer, par le dispositif électronique, un lissage sur les points de données dans la zone de troisième type en utilisant une pluralité de fenêtres de longueur fixe, pour obtenir des troisièmes données, la longueur de la fenêtre fixe utilisée pour effectuer le lissage dans la zone de troisième type étant inférieure à la longueur de la fenêtre fixe utilisée pour effectuer le lissage dans la zone de second type ;
effectuer, par le dispositif électronique, un lissage sur les points de données dans la zone de quatrième type en utilisant une pluralité de fenêtres de longueur non fixe, pour obtenir des quatrièmes données ; et
intégrer, par le dispositif électronique, les deuxièmes, troisièmes, quatrièmes données et les points de données dans la zone de premier type, afin d'obtenir les cinquièmes données obtenues après le lissage des données d'électrocardiogramme.

**2.** Procédé selon la revendication 1, dans lequel, lorsque la zone de quatrième type est une zone effectuant la transition de la zone de premier type vers la zone de troisième type, la longueur des fenêtres de longueur non fixe augmente progressivement au fil du temps ; ou
lorsque la zone de quatrième type est une zone effectuant la transition de la zone de troisième type vers la zone de premier type, la longueur des fenêtres de longueur non fixe diminue progressivement au fil du temps.

**3.** Le procédé selon la revendication 1 ou 2, dans lequel la première variabilité est liée à la détermination du point de données dans la zone de premier type, du point de données dans la zone de troisième type et du point de données dans la zone de quatrième type, et la division, par le dispositif électronique, des points de données dans les premières données en points de données dans une zone de premier type, des points de données dans une zone de deuxième type, des points de données dans une zone de troisième type et des points de données dans une zone de quatrième type comprend :

le calcul, par le dispositif électronique, de la première variabilité du point de données dans les premières données ; et
lorsque la première variabilité est inférieure à un premier seuil de variabilité, la détermination, par le dispositif électronique, que le point de données dans les premières données est le point de données dans la zone de deuxième type ; ou
lorsque la première variabilité n'est pas inférieure au premier seuil de variabilité, la détermination, par le dispositif électronique, que le point de données dans les premières données est le point de données dans la zone de premier type, le point de données dans la zone de troisième type ou le point de données dans la zone de quatrième type.

4.  Le procédé selon la revendication 3, dans lequel la seconde variabilité est liée à la détermination du point de données dans la zone de premier type, et la division, par le dispositif électronique, des points de données dans les premières données en points de données dans une zone de premier type, des points de données dans une zone de deuxième type, des points de données dans une zone de troisième type et des points de données dans une zone de quatrième type comprend en outre :

    le calcul, par le dispositif électronique, de la seconde variabilité du point de données dans la zone de premier type, du point de données dans la zone de troisième type ou du point de données dans la zone de quatrième type ; et lorsque la seconde variabilité n'est pas inférieure à un second seuil de variabilité, la détermination, par le dispositif électronique, que le point de données dans les premières données est le point de données dans la zone de premier type ; ou lorsque la seconde variabilité est inférieure au second seuil de variabilité, la détermination, par le dispositif électronique, que le point de données dans les premières données est le point de données dans la zone de troisième type ou le point de données dans la zone de quatrième type.

5.  Le procédé selon la revendication 4, dans lequel la première variabilité M1 satisfait la formule suivante :

    $M_1$=mean(abs(diff($X_1$)))/(2k+1), où k est un entier positif, une longueur de fenêtre choisie lors du calcul de $M_1$ est 2k+2, et $X_1$ est un ensemble de 2k+2 points de données dans les premières données dans une fenêtre ; et la seconde variabilité M2 satisfait la formule suivante :
    $M_2$=mean(abs(diff($X_2$)))/(2j+1), où j est un entier positif, une fenêtre choisie lors du calcul de $M_2$ est 2j+2, et $X_2$ est un ensemble de 2j+2 points de données dans les premières données dans la fenêtre.

6.  Procédé selon l'une quelconque des revendications 1 à 5, où $x_t^{r_2}$ est un point de données dans les deuxièmes données à un instant t, et le point de données $x_t^{r_2}$ dans les deuxièmes données satisfait la formule suivante :

    $$x_t^{r_2} = (x_{t-H}+\ldots+x_t+,\ldots,+x_{t+H})/(2H+1),$$

    où $x_t$ est un point de données dans les premières données à l'instant t, $x_{t-H}$ est un point de données dans les premières données à un instant t-H, $x_{t+H}$ est un point de données dans les premières données à un instant t+H, H est un entier positif et H est une constante, et une longueur de fenêtre sélectionnée lors du calcul de $x_t^{r_2}$ est 2H+1.

7.  Procédé selon l'une quelconque des revendications 1 à 5, où la longueur prédéfinie est q, $x_t^{r_3}$ est un point de données dans les troisièmes données à un instant t, et le point de données $x_t^{r_3}$ dans les troisièmes données satisfait la formule suivante :

    $$x_t^{r_3} = (x_{t-Q}+\ldots+x_t+,\ldots,+x_{t+Q})/(2Q+1),$$

    où $x_{t-Q}$ est un point de données dans les premières données à un instant t-Q, $x_{t+Q}$ est un point de données dans les premières données à un instant t+Q, Q est un entier positif et Q est une constante, Q<H, une longueur de fenêtre sélectionnée lors du calcul de $x_t^{r_3}$ est 2Q+1, et une plage de valeurs de la longueur de la fenêtre est [1, q].

8.  Procédé selon l'une quelconque des revendications 1 à 5, où $x_t^{r_4}$ est un point de données dans les quatrièmes données à un instant t, et le point de données $x_t^{r_4}$ dans les quatrièmes données satisfait la formule suivante :

    $$x_t^{r_4} = (x_{t-u}+\ldots+x_t+,\ldots,+x_{t+u})/(2u+1),$$

où $x_{t-u}$ est un point de données dans les premières données à un instant t-u, $x_{t+u}$ est un point de données dans les premières données à un instant t+u, u est un entier positif et u est une variable, et une longueur de fenêtre sélectionnée lors du calcul de $x_t^{r_4}$ est 2u+1.

9. Procédé selon l'une quelconque des revendications 1 à 8, où le procédé comprend en outre :
lorsque l'appareil électronique reçoit une opération de déclenchement utilisée pour démarrer la détection des données électrocardiographiques, afficher, par l'appareil électronique, les points de données dans les cinquièmes données.

10. Procédé selon l'une quelconque des revendications 1 à 9, où l'appareil électronique comprend un appareil portable, et l'appareil portable comprend un ou plusieurs des éléments suivants : une montre connectée, un bracelet connecté ou des lunettes intelligentes.

11. Appareil électronique, comprenant une mémoire, un processeur et un programme informatique stocké dans la mémoire et pouvant s'exécuter sur le processeur, dans lequel lorsque le processeur exécute le programme informatique, l'appareil électronique est apte à mettre en œuvre le procédé selon l'une quelconque des revendications 1 à 10.

12. Support de stockage lisible par ordinateur, dans lequel le support de stockage lisible par ordinateur stocke un programme informatique, et lorsque le programme informatique est exécuté par un processeur, un ordinateur est apte à mettre en œuvre le procédé selon l'une quelconque des revendications 1 à 10.

13. Produit programme informatique, comprenant un programme informatique, dans lequel lorsque le programme informatique est exécuté, un ordinateur est apte à mettre en œuvre le procédé selon l'une quelconque des revendications 1 à 10.

Electrocardiogram
measurement

a                    b

FIG. 1

Wearable device

Antenna 1    Antenna 2

| Mobile communication module<br>2G/3G/4G/5G<br>[150] | Wireless communication module<br>BT/WLAN/GNSS/NFC/IR/FM<br>[160] |

Speaker [170A]

Telephone receiver [170B]

Audio module [170]

Display screen [194]

Camera [193]

Indicator [192]

Key [190]

Internal memory [121]

Processor [110]

Sensor module [180]

Gyroscope sensor [180B]

Barometer [180C]

Magnetic sensor [180D]

Acceleration sensor [180E]

Optical proximity sensor [180G]

Temperature sensor [180J]

Touch sensor [180K]

Ambient light sensor [180L]

Charging management module [140]

Power management module [141]

FIG. 2

| Application layer | Watch face | Sports log | Phone | Exercise | | |
|---|---|---|---|---|---|---|
| System service layer | Step-counting service | Heart rate service | Calorie service | Heart health service | | |
| Algorithm layer | Heart rate algorithm | Dimming algorithm | Sleep algorithm | Wearing algorithm | | |
| Hardware abstraction layer | C++ library | Storage | Display | Touch control | BT | GPS |
| Kernel layer | OS kernel | | | | | |

FIG. 3

FIG. 4

EP 4 454 562 B1

An electronic device preprocesses electrocardiogram data to obtain first data —— S501

The electronic device divides data points in the first data into data points in a first-type area, data points in a second-type area, data points in a third-type area, and data points in a fourth-type area —— S502

The electronic device performs smoothing processing on the data points in the second-type area by using a plurality of fixed-length windows, to obtain second data —— S503

The electronic device performs smoothing processing on the data points in the third-type area by using a plurality of fixed-length windows, to obtain third data —— S504

The electronic device performs smoothing processing on the data points in the fourth-type area by using a plurality of non-fixed-length windows, to obtain fourth data —— S505

The electronic device integrates the second data, the third data, the fourth data, and the data points in the first-type area, to obtain fifth data obtained after the electrocardiogram data is smoothed —— S506

FIG. 5

a

b

FIG. 6

FIG. 7

FIG. 8

900

Electrocardiogram signal processing apparatus

Collection | Original electrocardiogram data 901

Preprocessing 902

High-pass filtering

Low-pass filtering → First data

903

Area division

First-type area

Second-type area

Third-type area

Fourth-type area

904

Smoothing processing

Not process

Second data

Third data

Fourth data

905

Data reconstruction | Fifth data

FIG. 9

Electronic device 1000

Display ⌐~1001

Processor ⌐~1002

Interface circuit ⌐~1003

Memory ⌐~1004

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210948840

- US 2013245478 A1

**Non-patent literature cited in the description**

- ECG signal denoising based on similar segments cooperative filtering. **LIU BAOYING et al.** BIOMEDICAL SIGNAL PROCESSING AND CONTROL. ELSEVIER, 19 May 2021, vol. 68